# EUROPÄISCHE PATENTSCHRIFT

(11) **EP 0 934 331 B1**
(45) Veröffentlichungstag und Bekanntmachung des Hinweises auf die Patenterteilung: **27.11.2002**
(21) Anmeldenummer: 97942899.2
(22) Anmeldetag: 29.08.1997
(51) Int. Cl.: C07H 21/00, C12Q 1/68

(54) **SPIEGELSELEKTION UND SPIEGELEVOLUTION VON NUCLEINSÄUREN**
MIRROR-SYMMETRICAL SELECTION AND EVOLUTION OF NUCLEIC ACIDS
SELECTION ET EVOLUTION SPECULAIRES D'ACIDES NUCLEIQUES

(30) Priorität: 30.08.1996 EP 96113953
(43) Veröffentlichungstag der Anmeldung: 11.08.1999
(73) Patentinhaber: Fürste, Jens Peter, 14057 Berlin (DE); Bald, Rolf, 10829 Berlin (DE); Erdmann, Volker A., 14163 Berlin (DE)
(72) Erfinder: Fürste, Jens Peter, 14057 Berlin (DE); Bald, Rolf, 10829 Berlin (DE); Erdmann, Volker A., 14163 Berlin (DE)
(74) Vertreter: VOSSIUS & PARTNER
(86) Internationale Anmeldenummer: EP9704726
(87) Internationale Veröffentlichungsnummer: WO98008856

(56) Entgegenhaltungen:
- WO-A-96/34879
- WO-A-97/43444
- SCHUMACHER T N M ET AL: "IDENTIFICATION OF D-PEPTIDE LIGANDS THROUGH MIRROR-IMAGE PHAGE DISPLAY" SCIENCE, Bd. 271, 29.März 1996, Seiten 1854-1857, XP000650829 in der Anmeldung erwähnt
- URATA ET AL.: "SYNTHESIS AND PROPERTIES OF MIRROR-IMAGE DNA" NUCLEIC ACID RESEARCH, Bd. 20, Nr. 13, 1992, Seiten 3325-3332, XP002055870 in der Anmeldung erwähnt
- MILTON DEL R C ET AL: "TOTAL CHEMICAL SYNTHESIS OF A D-ENZYME: THE ENANTIOMERS OF HIV-1 PROTEASE SHOW DEMONSTRATION OF RECIPROCAL CHIRAL SUBSTRATE SPECIFICITY" SCIENCE, Bd. 256, 5.Juni 1992, Seiten 1445-1448, XP000650852 in der Anmeldung erwähnt
- ELLINGTON A D ET AL: "IN VITRO SELECTION OF RNA MOLECULES THAT BIND SPECIFIC LIGANDS" NATURE, Bd. 346, 30.August 1990, Seiten 818-822, XP000673540
- LORSCH J R ET AL: "IN VITRO EVOLUTION OF NEW RIBOZYMES WITH POLYNUCLEOTIDE KINASE ACTIVITY" NATURE, Bd. 371, 1.September 1994, Seiten 31-36, XP002044711
- GOLD L: "OLIGONUCLEOTIDES AS RESEARCH, DIAGNOSTIC, AND THERAPEUTIC AGENTS" JOURNAL OF BIOLOGICAL CHEMISTRY, Bd. 270, Nr. 23, 9.Juni 1995, Seiten 13581-13584, XP002025263
- BOCK L C ET AL: "SELECTION OF SINGLE-STRANDED DNA MOLECULES THAT BIND AND INHIBIT HUMAN THROMBIN" NATURE, Bd. 355, Nr. 6360, 6.Februar 1992, Seiten 564-566, XP000453533
- NOLTE ET AL.: "MIRROR-DESIGN OF L-OLIGONUCLEOTIDE LIGANDS BINDING TO L-ARGININE" NATURE BIOTECHNOLOGY, Bd. 14, September 1996, Seiten 1116-1119, XP002039162
- KLUSSMANN ET AL.: "MIRROR-IMAGE RNA THAT BINDS D-ADENOSINE" NATURE BIOTECHNOLOGY, Bd. 14, September 1996, Seiten 1112-1115, XP002039163

## Beschreibung

Die Erfindung betrifft Verfahren zur identifizierung und Herstellung von L-Nucleinsäuren, die mit einem in der natürlichen Konfiguration auftretenden Zielmolekül in Wechselwirkung treten, sowie mit diesem Verfahren hergestellte L-Nucleinsäuren. Die Erfindung betrifft ferner die Verwendung von an die optische Antipode des Zielmoleküls bindenden D-Nucleinsäuren als Matrize zur Herstellung von L-Nucleinsäuren mit identischer Sequenz, sowie Arzneimittel, Kits, diagnostische Mittel und Sensorsysteme, welche die erfindungsgemäßen L-Nucleinsäuren enthalten.

In den letzten Jahren sind neue Technologien etabliert worden, die Nucleinsäuren in bisher nicht geahnter Weise zum Einsatz bringen können. Hierzu gehören z. B. der Einsatz dieser Moleküle als Katalysatoren, Inhibitoren oder Stimulatoren von biochemischen Reaktionen, die innerhalb oder außerhalb einer Zelle ablaufen. Es besteht kaum Zweifel, daß diese Technologien in der Zukunft eine dominante Rolle in den Bereichen der Medizin, pharmazeutischen Diagnostik, der Biotechnologie und der Landwirtschaft einnehmen werden.

Ein wesentlicher Bestandteil einiger der neuen DNA- und RNA-Technologien ist die Selektion bzw. Evolution *in vitro* (vgl. beispielsweise die Übersichtsartikel von J. W. Szostak, TIBS 17 (1992), 89 bis 93, Famulok und Szostak, Angew. Chemie 104 (1992), 1001-1011 und Gold *et al*., Annu. Rev. Biochem. (1995). Diese beruhen auf der Arbeitsweise biologischer Systeme. Dabei lassen sich aus einer kombinatorischen Bibliothek heterogener Nucleinsäuremoleküle durch Variation, Selektion und Replikation neue DNAoder RNA-Moleküle mit gewünschten Eigenschaften gewinnen. Damit sind in einem derartigen *in vitro*-System alle Faktoren gegeben, die auch bei der biologischen Evolution auftreten. Mit Recht kann daher hier von einer *in vitro*-Evolution gesprochen werden, die natürlicherweise ablaufende Vorgänge um ein Vielfaches beschleunigt. Sofern in diesem System keine zusätzlichen Variationsschritte auftreten, handelt es nicht um eine *in vitro*-Evolution, sondern lediglich um ein *in vitro*-Selektionsverfahren.

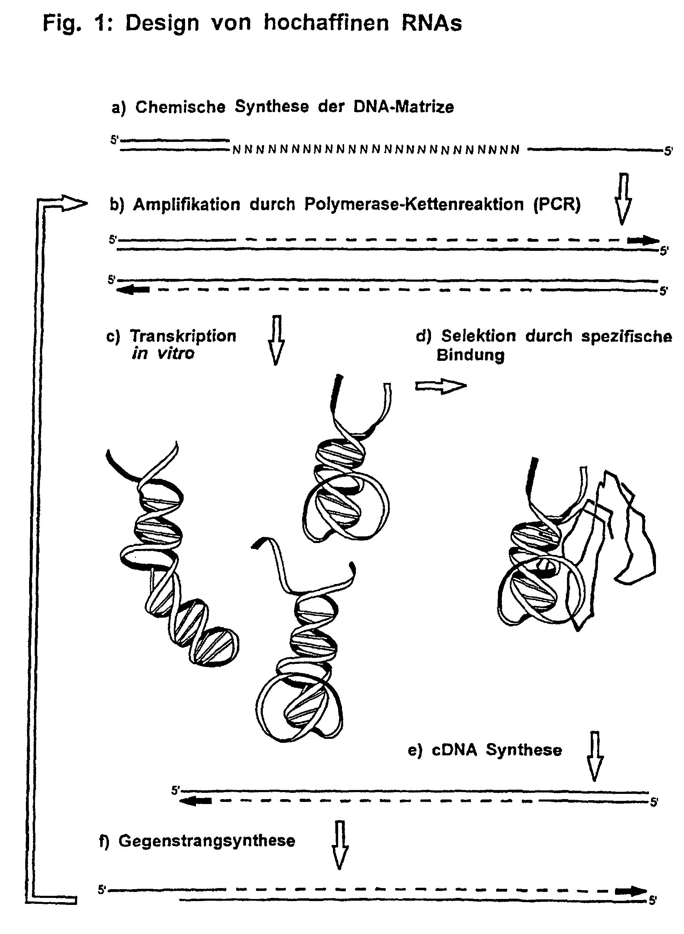

Aus einer Gruppe von bis zu 10¹⁸ verschiedenen RNA-Spezies können durch ein zyklisches Verfahren von Polymerase-Kettenreaktion (PCR). Transkription, selektiver Bindung und reverser Transkription die RNA-Moleküle isoliert werden, die hochaffine Bindungseigenschaften oder katalytische Eigenschaften besitzen: vgl. Gold et al., a.a.O.. Einer der wesentlichen Vorteile dieses Verfahrens liegt darin, daß bei dem zu selektionierenden Molekül keine Kenntnisse über die Struktur vorhanden sein müssen; Moleküle mit "richtiger" Struktur werden durch den Selektionsschritt aus der Ausgangspopulation an Molekülen herausgefiltert und können, falls erwünscht, anschließend sequenziert werden. Das Prinzip dieses Selektions- bzw. Evolutionsverfahrens ist in Fig. 1 schematisch dargestellt.

Beispiele für derartige *in vitro*-Selektions- bzw. -Evolutionsverfahren wurden von Tuerk und Gold, Science 249 (1990), 505-510, Berzal-Herranz et al., Genes & Development 6 (1992), 129-134 und von Robertson und Joyce. Nature 344 (1990), 467-468 bereitgestellt. Diese Arbeitsgruppen haben über etwas unterschiedliche Versuchsansätze erfolgreich funktionelle Ribonucleinsäuren selektioniert, die eine vorbestimmte. vom natürlichen Substrat verschiedene Nucleinsäure binden bzw. spalten. In einer neueren Arbeit konnten Lehmann und Joyce zeigen, daß sich die Metallionenspezifität eines Ribozyms von Mg2+ nach Ca2+ durch ein derartiges *in vitro*-Evolutionsverfahren ändern läßt (Nature 361 (1993), 182-185).

Hochaffine RNA- aber auch DNA-Moleküle können nicht nur zu dem Zweck konstruiert werden, daß sie mit anderen Nucleinsäuren, sondern vor allen Dingen mit Proteinen, anderen kleineren Molekülen der Zelle oder synthetischen Verbindungen in Wechselwirkung treten. Wechselwirkungen können aber auch mit den zellulären Rezeptoren bzw. mit viralen Partikeln angestrebt werden. In der Regel soll durch die Wechselwirkung der hochaffinen Nucleinsäure die Inhibierung oder Stimulierung einer biologischen Funktion oder in Sensorsystemen ein Signal erreicht werden.

Der Vorteil von Nucleinsäure-Bibliotheken gegenüber kombinatorischen Bibliotheken von anderen Oligomeren oder Polymeren besteht in der dualen Natur von Nucleinsäuren. Die Moleküle besitzen gleichzeitig Genotyp (vermehrungsfähige Sequenz) und Phänotyp (fünktionelle Struktur). Dadurch wird es möglich, aus sehr großen kombinatorischen Bibliotheken funktionelle Moleküle zu amplifizieren und durch Sequenzierung zu identifizieren. Die zusätzliche Markierung der Molekülbibliothek zur Identifizierung funktioneller Varianten durch z. B. "Tagging" (Janda, Proc. Natl. Acad. Sci. USA 91 (1994), 10779-10785) und die damit verbundenen technischen Probleme (Gold et al., a.a.O.; Gold, J. Biol. Chem. 270 (1995), 13581-13584) lassen sich vermeiden. Bei der Verwendung von kombinatorischen Phagenbibliotheken zur Identifizierung von Peptidmotiven (Scott und Smith, Science 249 (1990), 386-390, Devlin et al., Proc. Natl. Acad. Sci. USA (1990), 6378-6382), bei der die Verbindung von Genotyp und Phänotyp ebenfalls gegeben ist, bestehen andere Nachteile. Während Oligonucleotide von nur 25 Nucleotiden schon sehr stabile Strukturen ausbilden können, besitzen vergleichbare Oligopeptide große konformationelle Freiheiten (Gold et al., a.a.O.). Die strukturelle Freiheit von Peptiden und die daraus resultierenden entropischen Nachteile bei der Wechselwirkung mit Zielstrukturen limitieren die Einsatzmöglichkeiten von Peptiden, soweit hohe Affinitäten und Spezifitäten für die Anwendung der Moleküle benötigt werden. Diese Einschränkungen gelten auch für die Selektion biologisch stabiler D-Peptide durch Phagenbibliotheken (Schumacher et al., Science 271 (1996), 1854-1857). Auch die Verwendung cyclischer Peptide kann diese prinzipiellen Nachteile nicht aufheben (Gold et al., a.a.O.).

Der besondere Nachteil bei der Verwendung von kombinatorischen Nucleinsäure-Bibliotheken gegenüber anderen Oligomeren oder Polymeren besteht in der geringen Stabilität von Nucleinsäuren in biologischen Flüssigkeiten.

Alle bisher bekannten Selektions- und Evolutionsverfahren sind jedoch nur in der Lage, hochaffine oder katalytische RNAs bzw. DNAs in der natürlichen Form, d. h. mit D-Ribose bzw. D-Desoxyribose als Grundkörper herzustellen. Bei der Anwendung in biologischer Umgebung werden diese Moleküle von Enzymen abgebaut. Der Abbau führt zu einer kurzen Wirkungszeit dieser hochaffinen oder katalytischen Nucleinsäuren.

Zwar besteht die Möglichkeit, nach der Selektion von unmodifizierten Nucleinsäuren eine gezielte Modifikation einzuführen, die den enzymatischen Abbau verlangsamen soll. Allerdings läßt sich der Einfluß dieser Modifikation auf die Struktur und damit die Funktionsfähigkeit der Nucleinsäuren nicht vorhersagen. Weiterhin lassen sich veränderte, unerwünschte Eigenschaften nicht abschätzen. Zudem filhrt der Abbau chemisch modifizierter DNAs oder RNAs zu Produkten, die als Analoga von Nucleosiden, Nucleotiden oder Oligonucleotiden auf schwerwiegende und nachteilige Weise in den Zellmetabolismus eingreifen können.

Weiterhin ist es möglich, in das Verfahren modifizierte Nucleosidtriphosphate einzubeziehen, die die Stabilität der Nucleinsäuren erhöhen. Beispiele für diese Vorgehensweise wurden von Jellinek et al., Biochemistry 34 (1995), 11363-11372 und Eaton and Pieken, Annu. Rev. Biochem. 64 (1995) 837-863 beschrieben. Da die Nuclosidtriphosphate mit den verwendeten Polymerasen kompatibel sein müssen, ist das Spektrurn der möglichen Modifikationen sehr begrenzt. Weiterhin ist zu erwarten, daß der Abbau dieser modifizierten Nucleinsäuren zu besonders toxischen Effekten führt.

Der Erfindung lag somit das technische Problem zugrunde, Verfahren bereitzustellen, mit denen über *in vitro*-Selektion bzw. -Evolution hochaffine Nucleinsäuremoleküle hergestellt werden können, die die vorstehend genannten, im Stand der Technik bekannten Nachteile nicht aufweisen. Dieses technische Problem wird durch die in den Ansprüchen gekennzeichneten Ausführungsformen gelöst. Die Erfindung betrifft somit ein Verfahren zur Herstellung von L-Nucleinsäuren mit einer Mindestlänge von 15 Nucleotiden endhaltend L-Adenosin, L-Cytidin, L-Guanosin und L-Uridin, welche mit einem in der natürlichen Konfiguration auftretenden Zielmolekül in Wechselwirkung treten, das die folgenden Schritte umfaßt:
(a) Erzeugung einer heterogenen Population von D-Nucleinsäuren;
(b) Inkontaktbringen der Population von Schritt (a) mit der optischen Antipode des Zielmoleküls;
(c) Abtrennen der D-Nucleinsäuren, die nicht mit der optischen Antipode des Zielmoleküls in Wechselwirkung getreten sind;
(d) Sequenzierung der D-Nucleinsäuren, die mit der optischen Antipode des Zielmoleküls in Wechselwirkung getreten sind;
(e) Synthese von L-Nucleinsäuren, die in ihrer Sequenz mit den in Schritt (d) für die D-Nucleinsäuren ermittelten Sequenzen identisch sind.

Unter "L-Nucleinsäure" wird hier jede Nucleinsäure verstanden, die in der natürlicherweise nicht vorkommenden L-Konf guration vorkommt. Dies bedeutet, daß anstelle der natürlicherweise das Rückgrat einer Nucleinsäure bildenden D-Ribose oder D-Desoxyribose als Grundkörper der L-Nucleinsäuren L-Ribose bzw. L-Desoxyribose verwendet werden.

Unter dem Begriff "in der natürlichen Konfiguration auftretendes Zielmolekül" wird jedes mögliche Molekül verstanden, an das eine Nucleinsäure binden kann, sofern es in seiner in der Natur auftretenden Struktur vorkommt. Beispiele für solche Moleküle sind aus L-Aminosäuren zusammengesetzte Proteine, L-Aminosäuren, aus D-Nucleotiden bestehende Nucleinsäuren, sowie D-Zucker und daraus zusammengesetzte komplexere Zuckermoleküle.

Die Erzeugung einer heterogenen Population von D-Nucleinsäuren kann mit jedem im Stand der Technik bekannten Verfahren erfolgen. Beispiele für derartige Verfahren sind die Amplifikation genomischer Fragmente (Kinzler und Vogelstein, Nucl. Acids Res. 17 (1989), 3645-3635) oder die chemische Festphasensynthese von DNA-Molekülen an Syntheseautomaten (Thiesen und Bach, Nucl. Acids Res. 18 (1990), 3203-3209 und Pollock und Treisman, Nucleic Acids Res. 18 (1990). 6197-6204). Darüber hinaus ist der Fachmann in der Lage, je nach Versuchsanordnung Abwandlungen dieser Verfahren bereitzustellen, die ebenfalls zum gewünschten Ergebnis führen. Die Nucleinsäuren können aus einer beliebigen Zahl von D-Nucleotiden zusammengesetzt sein. Vorzugsweise weisen die D-Nucleinsäuren in mindestens 15 Positionen zufällige (nicht vorbestimmte) Nukleotide auf.

Die heterogene Population von D-Nucleinsäuren weist eine beliebige Anzahl von Mitgliedem, vorzugsweise mindestens 10⁹ Mitglieder auf.

Die D-Nucleinsäuren werden in Schritt (b) mit der optischen Antipode des Zielmoleküls in Kontakt gebracht, die eine Wechselwirkung der (hoch)affinen Nucleinsäure mit der optischen Antipode des Zielmoleküls erlauben.

Unter "optischer Antipode des Zielmoleküls" wird hier die enantiomere Form eines in der natürlichen Konfiguration vorkommenden (Makro)Moleküls verstanden. Die optische Antipode des Zielmoleküls kann nach dem im Stand der Technik beschriebenen Verfahren hergestellt werden. So haben beispielsweise Orata et al. (Nucl. Acids Res. 20 (1992), 3325 bis 3332) die Synthese eines aus L-Desoxyribosen zusammengesetzten Hexadesoxyribonucleotids beschrieben. Ferner können die L-Nucleinsäuren, wie in Beispiel 1 beschrieben, hergestellt werden. Die optische Antipode eines L-(Poly)peptids kann zum Beispiel nach den von Milton et al. (Science 258 (1992), 1445-1448) oder den von Muir (Structure 3 (1995), 649-652) beschriebenen Verfahren von hergestellt werden.

Die Abtrennung der D-Nucleinsäuren, die nicht mit dem Zielmolekül in Wechselwirkung getreten sind, erfolgt nach im Stand der Technik bekannten Verfahren. Beispielsweise kann die Abtrennung über ein säulenchromatographisches Verfahren erfolgen, wobei die optische Antipode des Zielmoleküls an das Säulenmaterial gebunden ist und affine bzw. hochaffine D-Nucleinsäuren unter geeigneten Bedingungen zurückgehalten werden. Die so gebundenen Nucleinsäuren können nach Auswaschen der nicht-gebundenen Nucleinsäuren vom Säulenmaterial eluiert werden. Die Abtrennung kann aber auch über Trenntechniken wie Filtermethoden oder magnetische Partikel erfolgen. Der Fachmann ist darüber hinaus in der Lage, die im Stand der Technik bekannten Verfahren für seine speziellen Bedürfnisse zu modifizieren.

Nachdem die nicht-wechselwirkenden Nucleinsäuren von den wechselwirkenden getrennt sind, werden die wechselwirkenden Nucleinsäuren von der optischen Antipode des Zielmoleküls getrennt. Sofern eine limitierte Heterogenität der Population von Schritt (a) vorgelegen hat und davon ausgegangen werden kann, daß eine genügende Anzahl gleicher oder ähnlicher Moleküle vorliegt, können die zuvor mit der optischen Antipode des Zielmoleküls wechselwirkenden D-Nucleinsäuren direkt sequenziert werden. Geeignete Sequenzierverfahren sind im Stand der Technik bekannt; vgl. Sambrook et al., Molecular Cloning, A Laboratory Manual 2. Aufl. 1989, Cold Spring Harbor Laboratory, Cold Spring Harbor. Sofern in diesem Selektionsverfahren Nucleinsäuren verschiedener Sequenz an die optische Antipode des Zielmoleküls binden und anschließend sequenziert werden, kann eine Sequenzinformation erhalten werden, die an verschiedenen Positionen mehrdeutig ist. Allerdings kann damit gerechnet werden, daß eine Anzahl von Nucleinsäuren in bestimmten Positionen konserviert ist. Dies deutet auf ihre Rolle bei der Bindung der optischen Antipode des Zielmoleküls hin, wie von Blackwell et al. gezeigt werden konnte (Blackwell und Weintraub, Science 250 (1990), 1104 bis 1110, Blackwell et al., Science 250 (1990), 1149 bis 1151). Mit dieser limitierten Information ist der Fachmann bereits in der Lage, eine L-Nucleinsäure zu synthetisieren, die an das gewünschte Zielmolekül binden kann.

Das Verfahren zur Identifizierung spiegelbildlicher Nucleinsäuren ist für viele Anwendungen aufwendig, da zunächst die optische Antipode der Zielstruktur in enantiomerenreiner Form zu präparieren ist. Die Racemat-Spaltung läßt sich umgehen, wenn die enantiomerenreine Zielstruktur zur Verfügung steht. In einer weiteren bevorzugten Ausführungsform betrifft die Erfindung daher ein Verfahren, das die D-Nucleinsäuren mit dem racemischen Gemisch eines Zielmoleküls in Wechselwirkung bringt. Die Abtrennung der D-Nucleinsäuren, die nicht mit dem racemischen Gemisch des Zielmoleküls in Wechselwirkung getreten sind, erfolgt nach im Stand der Technik bekannten Verfahren. Beispielsweise kann die Abtrennung über ein säulenchromatographisches Verfahren erfolgen, wobei das racemische Gemisch des Zielmoleküls an das Säulenmaterial gebunden ist, und affine bzw. hochaffine D-Nucleinsäuren unter geeigneten Bedingungen zurückgehalten werden. Hier binden zunächst Nucleinsäuren. die entweder (a) an die Zielstruktur, (b) an die spiegelbildliche Zielstruktur oder (c) aufgrund geringer chiraler Diskriminierung mit beiden Isomeren in Wechselwirkung treten können. Der Anteil hochaffiner D-Nucleinsäuren, der an das natürliche Enantiomer gebunden ist oder geringe chirale Spezifität besitzt, kann durch Elution mit dem natürlichen Zielmolekül ausgewaschen werden. Die verbliebenen, an die optische Antipode gebundenen D-Nucleinsauren, können dann mit dem racemischen Gemisch spezifisch eluiert werden. Damit wird es möglich, das Verfahren der Spiegeiselektion bzw. Spiegelevolution auch ohne Isolierung der optischen Antipode des Zielmoleküls durchzuführen.

Die Synthese der L-Nucleinsäuren erfolgt nach dem im Stand der Technik bekannten Verfahren (Urata et al., a.a.O.) bzw. nach dem in Beispiel 1 beschriebenen Verfahren.

Aus den Betrachtungen von L. Pasteur (z B. in Soc. Chim. Paris 1860, 1, (1860) über Enantiomere läßt sich postulieren, daß die natürlich auftretenden Nucleinsäuren optische Antipoden besitzen, deren Grundkörper die L-Ribose bzw. L-Desoxyribose ist. Erfindungsgemäß ist nunmehr gelungen, die chemische Festphasensynthese von L-RNA und L-DNA in beliebiger Sequenz zu etablieren (vgl. Beispiel 1).

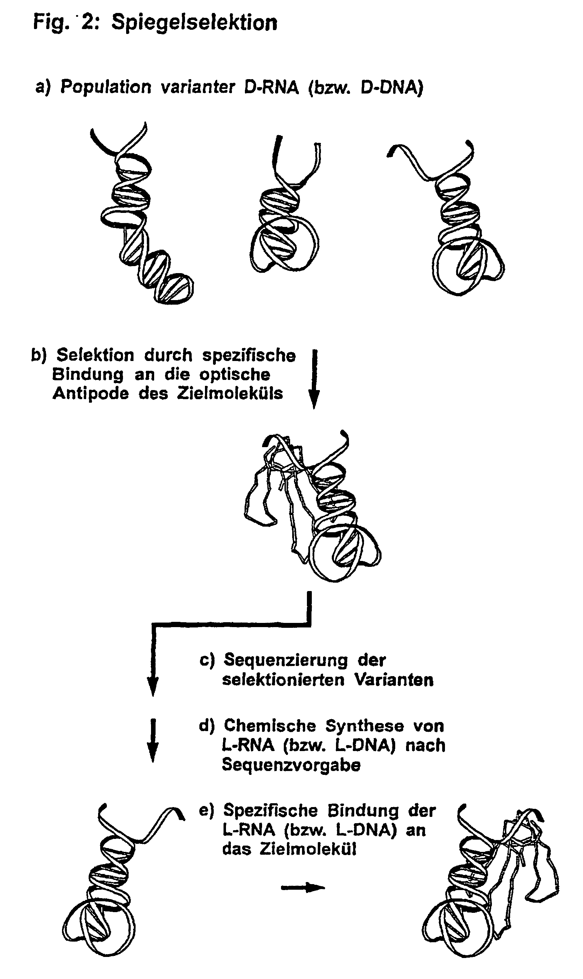

Betrachtet man jetzt eine nach dem erfindungsgemäßen Verfahren selektionierte Nucleinsäure, so läßt sich postulieren, daß ein entsprechendes L-Polymeres identischer Sequenz mit der optischen Antipode des Zielmoleküls in gleicher Weise wechselwirkt. Wird nun das Enantiomere des Zielmoleküls bei dem Evolutions- oder Selektionsverfahren eingesetzt, so läßt sich eine Sequenzinformation gewinnen, die die Produktion von hochaffinen oder katalytischen L-RNAs bzw. L-DNAs erlaubt. Das Prinzip dieses Verfahrens ist in Fig. 2 dargestellt. Der Vorteil dieses als Spiegelselektion oder Spiegelevolution bezeichneten Verfahrens liegt in der hohen Stabilität der Produkte in biologischer Umgebung. Mit diesem Verfahren wurde erstmalig ein L-Oligoribonucleotid identifiziert, das mit hoher Affinität an das natürlich auftretende D-Adenosin bindet (vgl. Beispiel 2). Nach Inkubation des L-Oligoribonucleotids in Humanserum konnte kein Abbau festgestellt werden. Bei den nachfolgenden Untersuchungen ließ sich nachweisen, daß das L-Oligoribonucleotid nur das D-Adenosin bindet und das D-Oligoribonucleotid nur das L-Adenosin. Damit ist gezeigt, daß sich D- und L-Oligonucleotide mit identischen, kovalent verknüpften Nucleotidbindungen in exakt gleicher Weise zur dreidimensionalen Struktur falten, und daß die Nucleotidsequenz allein die Tertiärstruktur einer Nucleinsäure bestimmt. Die in Beispiel 2 beschriebene Affinität der L-RNA zu D-Adenosin (Dissoziationskonstante von 1,8 µM) liegt im Bereich der bisher durch *in vitro* Evolution erzielten Affinitäten von D-RNA zu Nucleosiden (vgl. Connell und Yarus, Science 264 (1994), 1137-1141 und Huizenga und Szostak, Biochernistry 34 (1995), 656-665). Damit ist erstmalig gezeigt, daß L-Nucleinsäuren in der Lage sind, gleichgroße Affinitäten zu natürlich auftretenden chiralen Zielsubstanzen zu entwickeln, wie D-Nucleinsäuren. Das Prinzip des Verfahrens konnte durch die Identifizierung von Arginin-spezifischen Liganden bestätigt werden (vgl. Beispiel 3).

Die Spiegelevolution bzw. Spiegelselektion weist die Besonderheit auf, daß auf die optische Antipode des Zielmoleküls selektioniert wird. Diese Besonderheit bringt zwei wesentliche Vorteile mit sich: Zum einen wird die optische Antipode des Zielmoleküls während des erfindungsgemäßen Verfahrens nicht so leicht abgebaut, wie dies für die natürlich vorkommenden Enantiomere der Fall ist. Dies spielt insbesondere dann eine Rolle, wenn das Zielmolekül eine RNA ist. Zweitens bietet das hier beschriebene Verfahren insbesondere den im Stand der Technik unbekannten Vorteil, daß nach erfolgter Selektion bzw. abgeschlossener Evolution das Enantiomere der selektionierten (hoch)affinen D-Nucleinsäure hergestellt wird.

Diese L-Nucleinsäure weist nicht nur die vorstehend beschriebene hohe Stabilität in biologischer Umgebung auf, die ihre langanhaltende Wirkung in biologischen Systemen garantiert. Als Folge dieser Stabilität, die auf das Fehlen geeigneter degradierender Enzyme in biologischen Systemen zurückzurühren ist, entstehen keine Metaboliten dieser Nucleinsäuren, die eine gesundheitliche Gefährdung darstellen könnten. Darüber hinaus dürften diese L-Nucleinsäuren auch vom Immunsystem nicht oder kaum prozessiert werden und damit, wenn überhaupt, eine nur geringe Immunreaktion hervorrufen. Die Produkte des erfindungsgemäßen Verfahrens können demnach unbedenklich als Arzneimittel eingesetzt werden.

Das erfindungsgemäße Verfahren liefert somit Produkte, die Spiegelbilder der natürlich vorkommenden Nucleinsäuren sind. Diese Produkte haben eine definierte Sequenz und besitzen eine hohe Bindungsaffinität gegen einen vorgegebenen Liganden oder katalysieren eine gewünschte Reaktion an einem beliebigen Zielmolekül. Damit verfügt man über chemisch synthetisierte Polymere, die eine hohe biologische Stabilität aufweisen und in Analogie zu monoclonalen Antikörpern eingesetzt werden können. Die vielseitigen hydrolytischen Eigenschaften, aber möglicherweise auch synthetischen Eigenschaften, machen diese erfindungsgemäßen Moleküle, insbesondere für chemische oder pharmazeutische Anwendungen interessant.

In einer weiteren bevorzugten Ausführungsform betrifft die Erfindung ein Verfahren, wobei zusätzlich im Anschluß an Schritt (c) folgender Schritt eingefügt wird:
(ca) Amplifikation der D-Nucleinsäuren, die mit der optischen Antipode des Zielmoleküls in Wechselwirkung getreten sind.

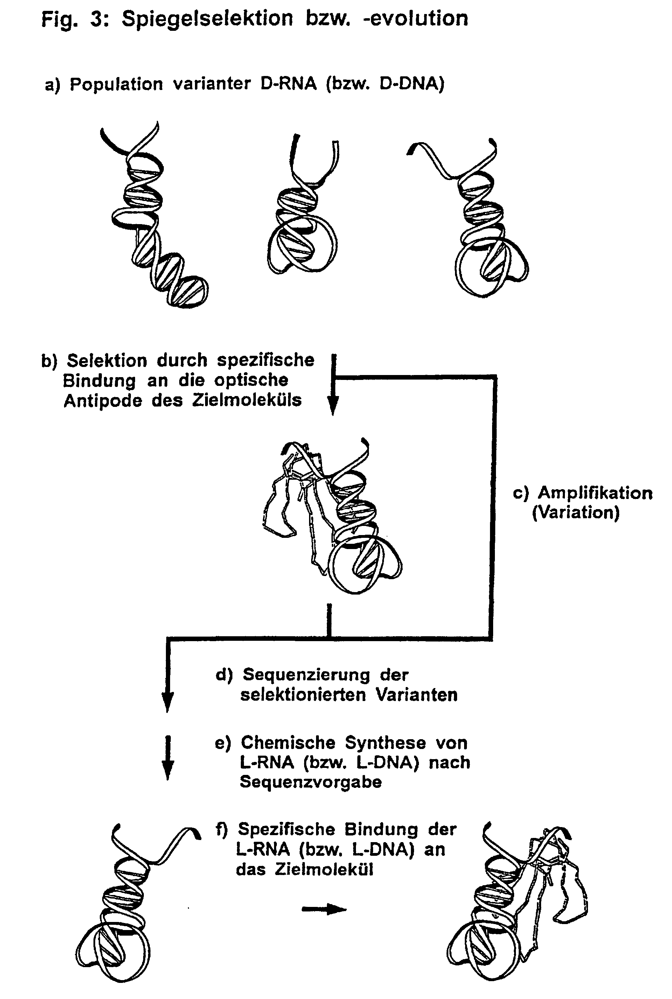

Mit dieser Ausführungsform des erfindungsgemäßen Verfahrens lassen sich insbesondere solche D-Nucleinsäuren selektionieren, die in der heterogenen Ausgangspopulation in geringer Anzahl vorliegen. Das Prinzip dieser bevorzugten Ausführungsform ist in Fig. 3 dargestellt. Der zusätzliche Amplifikationsschritt vermehrt die durch Bindung an die optische Antipode des Zielmoleküls erhaltenen (hoch)affinen D-Nucleinsäuren. Diese können in einem erneuten Selektionsschritt weiter angereichert oder nach Amplifikation direkt sequenziert werden.

Die Amplifikationen können jedoch auch in isothermalen Systemen durchgeführt werden. Derartige Systeme wurden beispielsweise von Guatelli et al. (Proc. Natl. Acad. Sci. USA 87 (1990) 1874-1878), und Walker et al. (Nucl. Acids Res. 20 (1992), 1691-1696) beschrieben.

In einer weiteren bevorzugten Ausführungsform des erfindungsgemäßen Verfahrens weisen die D-Nucleinsäuren der Population von Schritt (a) an ihren 5'- und 3'-Enden Primerbindungsstellen bzw. Komplementärsequenzen zu Primerbindungsstellen auf, die eine Amplifikation der in Schritt (ca) erhaltenen D-Nucleinsäuren durch PCR ermöglichen.

Diese Ausführungsform des erfindungsgemäßen Verfahrens ist besonders geeignet, wenn die Selektion der D-Nucleinsäure auf RNA-Ebene erfolgt. Darüber hinaus kann die Transkription mittels einer DNA-abhängigen RNA Polymerase als zusätzlicher Amplifikationsschritt neben der PCR verwendet werden. Eine Kombination dieser beiden Amplifikationsschritte ermöglicht somit eine besonders hohe Ausbeute an D-Nucleinsäurematerial, das mit der optischen Antipode des Zielmoleküls in Wechselwirkung getreten ist.

In einer anderen bevorzugten Ausführungsform des erfindungsgemäßen Verfahrens wird im Anschluß an Schritt (ca) folgender Schritt eingefügt:
(cb) Inkontaktbringen der amplifizierten D-Nucleinsäuren mit der optischen Antipode des Zielmoleküls,
an die sich die Schritte (b) und gegebenenfalls (ca) vor der Durchführung des Schrittes (d) anschließen, wobei die Schritte (cb), (b) und gegebenenfalls (ca) in dieser Reihenfolge ein oder mehrere Male wiederholt werden können.

Die Amplifikation der in den Selektionsschritten isolierten D-Nucleinsäuren durch PCR führt auf bequeme Weise zur Anreicherung der gewünschten D-Nucleinsäuren. In der bevorzugten Ausführungsform des erfindungsgemäßen Verfahrens, bei dem mehrere Cyclen von Inkontaktbringen der amplifizierten D-Nucleinsäuren mit der optischen Antipode des Zielmoleküls, nachfolgendes Abtrennen der nicht gebundenen Moleküle (Selektion) und Amplifikation aufeinander folgen, werden letztendlich die Nucleinsäuren mit der höchsten Bindungsaffinität selektioniert. Durch Variation des molaren Verhältnisses von optischer Antipode des Zielmoleküls und D-Nucleinsäuren kann auf eine Mehrzahl von (hoch)affinen D-Nucleinsäuren (Verhältnis >1) oder auf eine oder nur wenige Nucleinsäuren (Verhältnis ≤1) selektioniert werden. Entsprechend erhält man in Schritt (e) eine oder wenige hochaffine L-Nucleinsäuren oder eine Mehrzahl (hoch)affiner L-Nucleinsäuren. Ein entsprechendes Ergebnis läßt sich durch Variation der Anzahl von Selektionsschritten erreichen, wobei eine große Anzahl von Selektionsschritten letztendlich auf eine oder wenige D-Nucleinsäuren mit hohen Bindungsaffinitäten selektionieren wird.

Der Amplifikationsschritt in dem erfindungsgemäßen Verfahren läßt sich bequemerweise durch PCR bewerkstelligen. Die PCR ist ein im Stand der Technik gut etabliertes Verfahren, dessen Prinzipien beispielsweise in Sambrook, a.a.O. beschrieben sind. Sowohl RNA als auch DNA können über PCR amplifiziert werden, wobei bei der Amplifikation von RNA noch ein Verfahrensschritt, der die Umschreibung von RNA in eine korrespondierende cDNA mittels reverser Transkriptase bewirkt, eingefügt werden kann.

Die Amplifikation kann jedoch auch mit anderen im Stand der Technik bekannten Verfahren erzielt werden.

In einer weiteren bevorzugten Ausführungsform des erfindungsgemäßen Verfahrens werden bei der Amplifikation Nucleotide in die neu zu synthetisierenden Nucleotidstränge eingebaut, die an dieser Nucleotidsäureposition in den in Schritt (a) vorkommenden, mit dem Zielmolekül wechselwirkenden D-Nucleinsäuren nicht vorkommen.

Durch diese bevorzugte Ausführungsform kann ähnlich wie in der biologischen *in vivo*-Evolution auf neue, in der Ausgangspopulation nicht vorkommende D-Nucleinsäurespecies selektioniert werden. Hier liegt also ein Fall von *in vitro*-Evolution vor. Mehrere Varianten dieses Verfahrens sind denkbar, die alle in den Schutzbereich der vorliegenden Erfindung fallen.

Einmal kann die Amplifikation per se durch ein Verfahren erfolgen, das eine gewisse Fehlerrate beim Einbau der Nucleotide in den neu zu synthetisierenden Strang aufweist. Die PCR ist als ein solches Verfahren bekannt.

Sofern die für eine optimale Binding erforderliche Bindungsstelle mehr als etwa 25 Nucleotide aufweist, ist die entsprechende Sequenz mit einer gewissen Wahrscheinlichkeit nicht in der ursprünglichen Population von Schritt (a) enthalten. Dies liegt daran, daß aus Praktikabilitätsgründen ab einer bestimmten Länge der Nucleotidsequenz (die im Bereich von etwa 25 Nucleotiden liegt) in dieser Population nicht jede mögliche Sequenz enthalten sein kann.

Wenn die optimale Sequenz für die Bindung an die optische Antipode des Zielmoleküls in der Population von Schritt (a) tatsächlich nicht vorhanden ist, kann sie dennoch mit dem eᵣfindungsgemäßen Verfahren selektioniert werden. Dies geschieht dadurch, daß zunächst eine D-Nucleinsäure mit suboptimaler Bindungsaffinität aus der Population isoliert wird (innerhalb der Population stellt diese Sequenz allerdings das Molekül mit optimaler Bindungsaffinität dar). Anschließend wird diese Sequenz während der Amplifikation mutagenisiert. Solche Mutagenisierungsverfahren sind im Stand der Technik bekannt (vgl. beispielsweise Light and Lernen, Bioorg. Med. Chem. 3 (1995), 995-967 und Pannekoek et al., Gene 128 (1993), 135-140). Dabei kann die gesamte Sequenz mutagenisiert werden. Die mutagenisierte Sequenz wird weiteren Selektionsschritten unterworfen, wobei mehrere Cyclen von Amplifikation mit Mutagenese und anschließende Selektion aneinandergereiht werden können, bis eine D-Nucleinsäure mit optimalen Bindungseigenschaften gefunden worden ist.

Ferner kann aus praktischen Gründen zunächst eine kurze D-Nucleinsäure eingefügt werden, die die optimalen Bindungseigenschaften ihrer Population aufweist. Man bestimmt nach im Stand der Technik bekannten Verfahren die für die Bindung essentiellen Positionen und ersetzt die übrigen Nucleotidbereiche durch längere Abschnitte. Daran schließen sich wiederum eine oder mehrere Amplifikations- und Selelektionsrunden an. Beim so ermittelten Molekül mit den optimalen Bindungseigenschaften werden wiederum die für die Bindung nicht essentiellen Teile der Sequenz durch eine randomisierte Sequenz ersetzt. Dieser Verfahrensabschnitt ist beispielsweise in W 91/19813 in anderem Zusammenhang beschrieben und wird dort als "Walking" bezeichnet.

In einer weiteren Ausführungsform der vorliegenden Erfindung werden L-Nucleinsäuren direkt mit einem in der natürlichen Konfiguration vorkommenden Zielmolekül in Wechselwirkung gebracht. Die Erfindung betrifft somit ein Verfahren zur Herstellung von L-Nucleinsäuren mit einer Mindestlänge von 15 Nucleotiden enthaltend L-Adenosin L-Cytidin, L-Guanosin und-L-Uridin, daß die folgenden Schritte umfaßt:
(a) Erzeugung einer heterogenen Population von L-Nucleinsäuren;
(b) Inkontaktbringen der Population von Schritt (a) mit dem Zielmolekül;
(c) Abtrennen der L-Nucleinsäuren, die nicht mit dem Zielmolekül in Wechselwirkung getreten sind;
(d) Sequenzierung der L-Nucleinsäuren, die mit dem Zielmolekül in Wechselwirkung getreten sind;
(e) Synthese von L-Nucleinsäuren, die in ihrer Sequenz mit den in Schritt (d) ermittelten Sequenzen identisch sind.

Die Erzeugung einer heterogenen Population von L-Nucleinsäuren erfolgt nach dem im Stand der Technik bekannten Verfahren (Urata et al., a.a.O.) bzw. nach dem in Beispiel 1 beschriebenen Verfahren. Nachdem die nicht wechelwirkenden L-Nucleinsäuren von den wechselwirkenden getrennt sind, werden die wechselwirkenden Nucleinsäuren vom Zielmolekül getrennt. Die L-Nucleinsäuren werden dann nach dem in Beispiel 4 aufgeführten Verfahren iterativ vereinzelt. Anstelle der in Beispiel 4 benutzten L-Proteine werden hier D-Proteine verwendet. Die optischen Antipoden der Enzyme können nach den von Milton et al. (a.a.O.) oder den von Muir (a.a.O.) beschriebenen Verfahren hergestellt werden. Die Strangtrennung kann mit der in Beispiel 4 beschriebenen Methode oder mit jedem im Stand der Technik bekanntem Verfahren durchgeführt werden. Beispiele für derartige Verfahren sind Strangtrennungsgele (Maxam und Gilbert, Proc. Natl. Acad. Sci. 78 (1977), 560-564, Maniatis, a.a.O.) oder Festphasensequenzierung (Hultman et al., Nucl. Acids Res. 17 (1989), 4937-4946, Hultman et al., BioTechniques 10 (1991), 84-93). Eine weitere Möglichkeit, einzelsträngige DNA nach einer PCR zu erhalten, besteht darin, einen Primer mit internem spacer (z.B. Polyethylenglykol) einzusetzen (Williams und Bartel, Nucleic Acids Res. 23, (1995) 4220-4221).
Die für die Sequenzierung benötigten L-Desoxynucleosidtriphosphate und L-Didesoxynucleosidtriphosphate werden durch chemische Synthese aus den in Beispiel 1 beschriebenen L-Nucleosiden nach den im Stand der Technik fur D-Nucleoside beschriebenen Verfahren gewonnen. Bei der Triphosphat-Darstellung werden zunächst die vier L-Desoxynucleoside an der 5'-Position phosphoryliert (Yoshikawa et al., Tetrahedron Lett. (50), 1967, 5065-5068). Die 5'-Monophosphate werden dann in ihre 5'-Triphosphate umgewandelt (Hoard und Ott, J. Am. Chem. Soc. 87 (1965), 1785-1788). Zur Darstellung der L-Didesoxynucleodidtriphosphate werden zunächst die L-Desoxynucleoside in L-Didesoxynucleoside umgewandelt. Die Synthese der Pyrimidin-L-didesoxynucleoside kann in mehrstufigen Verfahren nach Horwitz et al. (J. Org. Chem. 32 (1967), 817-818) und Joshi et al. (J. Chem. Soc. (1992), 2537-2544) erfolgen. Das Guanosin-Ldidesoxynucleosid ist nach Herdewijn et al. (J. Med. Chem. 31 (1988), 2040-2048), und das Adenosin-L-didesoxynucleosid ist nach Chu et al. (J. Org. Chem. 54 (1989), 2217-2225) darstellbar. Die erhaltenen vier L-Didesoxynucleoside werden dann mit den oben erwähnten Verfahren von Yoshikawa et al. (Tetrahedron Lett. (50), 1967. 5065-5068) und Hoard und Ott (Hoard und Ott. J. Am. Chem. Soc. 87 (1965), 1785-1788) über die Zwischenstufe der 5'-Monophosphate in ihre jeweiligen 5'-Triphosphate überführt. In einer weiteren bevorzugten Ausführungsform betrifft die Erfindung ein Verfahren, wobei zusätzlich im Anschluß an Schritt (c) folgender Schritt eingefügt wird:
(ca) Amplifikation der L-Nucleinsäuren, die mit dem Zielmolekül in Wechselwirkung getreten sind.

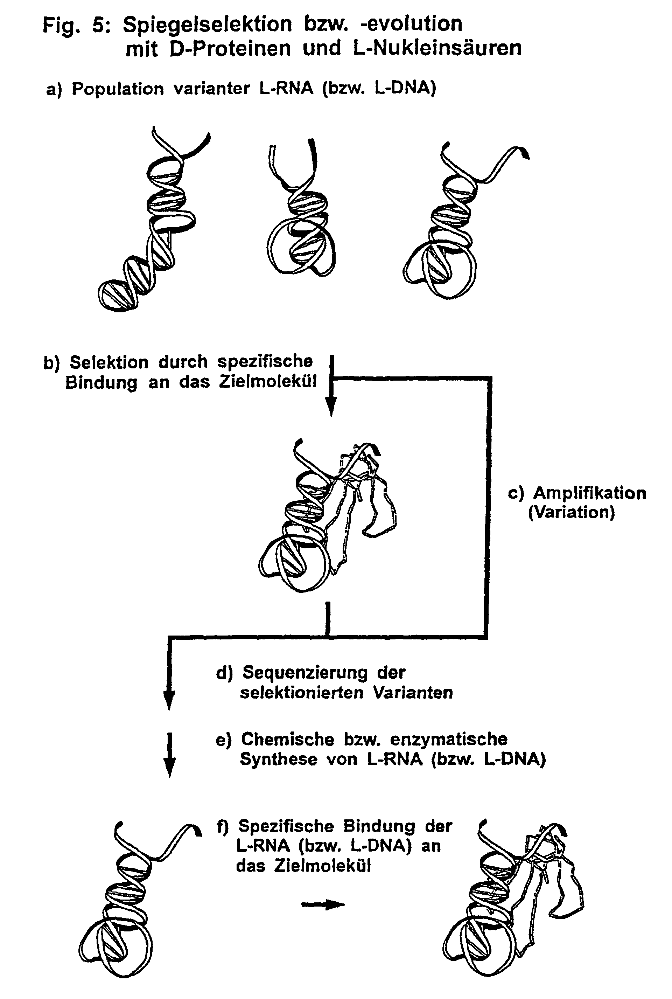

Die Vermehrung der L-Nucleinsäuren wird durch D-Polymerasen erreicht, die nach den von Milton et al. (a.a.O.) oder den von Muir (a.a.O.) beschriebenen Verfahren hergestellt werden. Mit dieser Ausführungsform des erfindungsgemäßen Verfahrens lassen sich insbesondere solche L-Nucleinsäuren selektionieren, die in der heterologen Ausgangspopulation in geringer Zahl vorliegen. Das Prinzip dieser bevorzugten Ausrührungsform ist in Fig. 5 dargestellt. Der zusätzliche Amplifikationsschritt vermehrt die durch Bindung an das Zielmolekül erhaltenen (hoch)affinen L-Nucleinsäuren. Diese können in einem erneuten Selektionsschritt weiter angereichert oder nach Amplifikation direkt sequenziert werden.

Die Amplifikationen können jedoch auch in isothermalen Systemen mit entsprechenden D-Polymerasen durchgeführt werden. Derartige Systeme wurden beispielsweise von Guatelli et al. (a.a.O.), und Walker et al. (a.a.O.) für L-Polymerasen beschrieben.

In einer weiteren bevorzugten Ausführungsform des erfindungsgemäßen Verfahrens weisen die L-Nucleinsäuren der Population von Schritt (a) an ihren 5'- und 3'-Enden Primerbindungsstellen bzw. Komplementärsequenzen zu Primerbindungsstellen auf, die eine Amplifikation der in Schritt (ca) erhaltenen L-Nucleinsäuren durch spiegelbildliche PCR ermöglichen.

Diese Ausführungsform des erfindungsgemäßen Verfahrens ist besonders geeignet, wenn die Selektion der L-Nucleinsäure auf RNA-Ebene erfolgt. Darüber hinaus kann die spiegelbildliche Transkription mittels einer DNA-abhängigen D-RNA Polymerase als zusätzlicher Amplifikationsschritt neben der spiegelbildlichen PCR verwendet werden. Eine Kombination dieser beiden Amplifikationsschritte ermöglicht somit eine besonders hohe Ausbeute an L-Nucleinsäurematerial, das mit dem Zielmolekül in Wechselwirkung getreten ist.

In einer anderen bevorzugten Ausführungsform des erfindungsgemäßen Verfahrens wird im Anschluß an Schritt (ca) folgender Schritt eingefügt:
(cb) Inkontaktbringen der amplifizierten L-Nucleinsäuren mit dem Zielmolekül, an die sich die Schritte (b) und gegebenenfalls (ca) vor der Durchführung des Schrittes (d) anschließen, wobei die Schritte (cb), (b) und gegebenenfalls (ca) in dieser Reihenfolge ein oder mehrere Male wiederholt werden können.

Die Amplifikation der in den Seiektionsschritten isolierten L-Nucleinsäuren durch spiegelbildliche PCR führt auf bequeme Weise zur Anreicherung der gewünschten L-Nucleinsäuren. In der bevorzugten Ausführungsform des erfindungsgemäßen Verfahrens, bei dem mehrere Cyclen von Inkontaktbringen der amplifizierten L-Nucleinsäuren mit dem Zielmolekül, nachfolgendes Abtrennen der nicht gebundenen Moleküle (Selektion) und Amplifikation aufeinander folgen, werden letztendlich die Nucleinsäuren mit der höchsten Bindungsaffinität selektioniert. Durch Variation des molaren Verhältnisses von Zielmolekül und L-Nucleinsäuren kann auf eine Mehrzahl von (hoch)affinen L-Nucleinsäuren (Verhältnis >1) oder auf eine oder nur wenige Nucleinsäuren (Verhältnis ≤1) selektioniert werden. Entsprechend erhält man in Schritt (e) eine oder wenige hochaffine L-Nucleinsäuren oder eine Mehrzahl (hoch)affiner L-Nucleinsäuren. Ein entsprechendes Ergebnis läßt sich durch Variation der Anzahl von Selektionsschritten erreichen, wobei eine große Anzahl von Selektionsschritten letztendlich auf eine oder wenige L-Nucleinsäuren mit hohen Bindungsaffinitaten selektionieren wird.

In einer weiteren bevorzugten Ausführungsform des crfindungsgemäßen Verfahrens werden bei der Amplifikation Nucleotide in die neu zu synthetisierenden Nucleotidstränge eingebaut, die an dieser Nucleotidsäureposition in den in Schritt (a) vorkommenden, mit dem Zielmolekül wechselwirkenden L-Nucleinsäuren nicht vorkommen.

Durch diese bevorzugte Ausführungsform kann ähnlich wie in der biologischen *in vivo*-Evolution auf neue, in der Ausgangspopulation nicht vorkommende L-Nucleinsäurespecies selektioniert werden. Hier liegt also ein Fall von spiegelbildlicher *in vitro*-Evolution vor. Mehrere Varianten dieses Verfahrens sind denkbar, die alle in den Schutzbereich der vorliegenden Erfindung fallen.

In einer bevorzugten Ausführungsform betrifft die Erfindung ein Verfahren, wobei die Wechselwirkung in einer Bindung besteht.

In einer weiteren bevorzugten Ausführungsform des erfindungsgemäßen Verfahrens besteht die Wechselwirkung in einer katalytischen Reaktion.

Da die an der Wechselwirkung beteiligten Moleküle vor Ablauf der katalytischen Reaktion miteinander in Wechselwirkung treten müssen. impliziert diese Ausführungsform ebenfalls eine Wechselwirkung z.B. der (hoch)affinen D-Nucleinsäure mit der optische Antipode des Zielmoleküls.

Derartige katalytische Reaktionen sind beispielsweise für Ribozyme beschrieben worden (vgl. beispielsweise Robertson und Joyce, a.a.O.). Beispiele für neuartige Katalysatore sind weiterhin von Pan und Uhlenbeck (Biochemistry 33 (1994), 9561-9565), sowie Bartel und Szostak (Science 261 (1993), 1411-1418) beschrieben worden. Lorsch und Szostak (Nature 371 (1994), 31-36) konten durch in vitro-Selektion ein Ribozym mit Kinase-Aktivität identifizieren. Breaker und Joyce (Chem. Biol. 1 (1994), 223-229) haben ein neues Desoxyribozym beschrieben, daß die Spaltung einer Phosphodiesterbindung katalysiert, während Cuenoud und Szostak (Nature 375 (1995), 611-614) ein DNA-Metalloenzym mit DNA-Ligase-Aktivität identifizieren konnten.

In einer anderen bevorzugten Ausführungsform der erfindungsgemäßen Verfahren sind die Nucleinsäuren von Schritt (a) Desoxyribonucleinsäuren.

In einer besonders bevorzugten Ausführungsform der erfindungsgemäßen Verfahren sind die Ribonucleinsäuren Desoxyribozyme.

In einer weiteren bevorzugten Ausführungsform der erfindungsgemäßen Verfahren sind die Nucleinsäuren Ribonucleinsäuren.

In einer besonders bevorzugten Ausrührungsform des erfindungsgemäßen Verfahrens sind die Ribonucleinsäuren Ribozyme.

Diese bevorzugte Ausführungsform des erfindungsgemäßen Verfahrens erlaubt die Selektion der gewünschten Moleküle nicht durch Bindung an die optische Antipode des Zielmoleküls, sondern auch deren katalytische Aktivität. Somit umfaßt Schritt (b) des erfindungsgemäßen Verfahrens unmittelbar in Zusammenhang mit der Bindung an die optische Antipode des Zielmoleküls gegebenenfalls auch eine Spaltung eines Substrats, das üblicherweise das Zielmolekül oder ein Teil davon sein wird. Die Spaltung des Substrats kann dabei Ausgangspunkt für die weitere Amplifikation und Selektion geeigneter Ribozyme sein. Entsprechende Systeme sind u. a. von Robertson und Joyce (a.a.O.) beschrieben worden.

In einer weiteren Ausführungsform des erfindungsgemäßen Verfahrens ist das Zielmolekül eine Aminosäure, ein Peptid, ein Polypeptid, oder ein aus mehreren Polypeptiden zusammengesetztes Protein. Die Polypeptide oder Proteine können glykosiliert oder nicht glykosiliert sein.

Erfindungsgemäß wird unter Aminosäure, Peptid. Polypeptid und Protein jedes dieser (Makro)Moleküle verstanden, mit der die D-Nucleinsäuren oder L-Nucleinsäuren (?) wechselwirken können. Beispiele derartiger (Makro)Moleküle sind Enzyme, Strukturproteine und Hormone. Die (Makro)Moleküle können demgemäß Bestandteile einer größeren Struktur sein oder frei in biologischen Systemen in Lösung vorliegen.

In einer anderen bevorzugten Ausführungsform des erfindungsgemäßen Verfahrens ist das Zielmolekül eine einzelsträngige RNA, eine doppelsträngige RNA, eine einzelsträngige DNA oder eine doppelsträngige DNA, sowie Kombinationen daraus.

Für den Fachmann ist selbstverständlich, daß diese Nucleinsäuren unter verschiedenen Bedingungen verschiedene Konformationen einnehmen können. Das erfindungsgemäße Verfahren umfaßt jede Konformation, die diese (Makro)Moleküle oder Kombinationen daraus einnehmen können. Das erfindungsgemäße Verfahren umfaßt weiterhin Kombinationen dieser Makromoleküle. Solche Kombinationen können beispielsweise aus einzelund doppelsträngiger RNA oder DNA oder aus Tripelhelices bestehen.

Ein Antibiotikum oder ein pharmazeutisch wirksames Substrat oder dessen Vorstufe ist das Zielmolekül einer weiteren bevorzugten Ausführungsform des erfindungsgemäßen Verfahrens.

Bevorzugte pharmazeutisch wirksame Substrate sind z. B. Steroide, ACE-Hemmer, β-Blocker und Diuretika. Mit den erfindungsgemäßen L-Nucleinsäuren kann somit wirksam in eine Therapie eingegriffen werden und die Halbwertszeit eines Antibiotikums oder pharmazeutisch wirksamen Substrats wirkungsvoll herabgesetzt werden.

Zielmolekül in einer weiteren bevorzugten Ausführungsform des erfindungsgemäßen Ver-fahrens ist ein Zuckermolekül, beispielsweise ein verzweigter oder ein unverzweigter Polyzucker.

Der Begriff "Zuckermolekül", wie hier verwendet, betrifft sowohl monomere als auch zusammengesetzte komplexe Zuckerstrukturen.

In einer anderen Ausführungsform des effindungsgemäßen Verfahrens erfolgt die Synthese der L-Nucleinsäure in Schritt (e) auf chemischem oder enzymatischem Wege.

In einer besonders bevorzugten Ausführungsform des erfindungsgemäßen Verfahrens unfaßt die chemische Synthese der L-Nucleinsäuren in Schritt (e) folgende Schritte.
(ea) Synthese von L-Nucleosiden;
(eb) Synthese von geschützten L-Nucleosidphosphoramiditen; und
(ec) Festphasensynthese der L-Nucleinsäuren am Automaten.

Die Erfindung betrifft ferner L-Nucleinsäuren mit einer Mindestlänge von 15 Nucleotiden enthaltend L-Adenosin, L-Cytidin, L-Guanosin und L-Uridin, die spezifisch an ein wie vorstehend definiertes Zielmolekül binden.

Die erfindungsgemäßen L-Nucleinsäuren werden vorzugsweise mit dem erfindungsgemäßen Verfahren hergestellt. Darüber hinaus sind die erfindungsgemäßen L-Nucleinsäuren mit jeder Abwandlung des erfindungsgemäßen Verfahrens herstellbar, sofern nur der Selektionsschritt unter Verwendung der optischen Antipode des Zielmoleküls und der Syntheseschritt unter Benutzung einer D-Nucleinsäure als Matrize verwendet werden.

Die erfindungsgemäßen L-Nucleinsäuren können vielfältig eingesetzt werden. Aufgrund der hohen Affinität für das Zielmolekül können sie in ähnlicher Weise wie monoclonale Antikörper eingesetzt werden. Sie können beispielsweise mit einem Marker oder einer cytotoxischen Gruppe versehen werden. Die erfindungsgemäßen L-Nucleinsäuren können zur Stimulierung oder zur Inhibierung der biologischen Funktion eines Zielmoleküls verwendet werden. Weiterhin lassen sich die L-Nucleinsäuren an Träger koppeln und als Affinitätsmaterial zur Reinigung oder Abtrennung von Zielmolekülen einsetzen. Immobilisierte L-Nucleinsäuren können z.B. zur Trennung von Enantiomeren oder zur Abtrennung enantiomerer Verunreinigungen eingesetzt werden. Sie können weiterhin zur Aufreinigung und/oder Abtrennung von zellulären Faktoren oder Zellen dienen. Nach Kenntnis von DNA-Sequenzen ist es z.B. möglich, aus den abgeleiteten Protein-Sequenzen entsprechende optische Antipoden (D-Proteine bzw. D-Peptide) im erfindungsgemäßen Verfahren einzusetzen und somit gezielt Affinitätsmaterialien herzustellen. Immobilisierte L-Nucleinsäuren können weiterhin als Affinitätsmaterial zur Abtrennung zellulärer Faktoren oder Zellen eingesetzt werden, wie z.B. bei der Abtrennung toxischer Komponenten durch Dialyse.

In einer besonders bevorzugten Ausführungsform ist die erfindungsgemäße L-Nucleinsäure eine L-Ribonucleinsäure. Diese L-Ribonucleinsäure kann in einzel- oder doppelsträngiger Form vorliegen.

In einer weiteren besonders bevorzugten Ausführungsform ist die erfindungsgemäße L-Nucleinsäure ein Ribozym.

Diese Erfindung beschreibt erstmalig die Herstellung eines hochaffinen L-Oligoribonucleotids bzw. L-Oligodesoxyribonucleotids (vgl. Beispiel 1). Die durch das Verfahren identifizierte L-RNA bindet spezifisch an das D-Adenosin (vgl. Beispiel 2) bzw. L-Arginin (vgl. Beispiel 3). In Vergleichsversuchen konnte gezeigt werden, daß die D-Form dieser hochaffinen L-Oligoribonucleotide die entsprechende enantiomere Form des Adenosins (vgl Beispiel 2) bzw. Arginins (vgl. Beispiel 3) bindet. Somit ist der Nachweis erbracht, daß die beiden enantiomeren Formen der hochaffinen Oligoribonucleotide sich in exakt gleicher Weise zur dreidimensionalen Struktur falten. Die Voraussagen von Pasteur (aa.O.) über die biologische Aktivität optischer Enantiomere konnte damit bestätigt werden.

Die Erfindung umfaßt ferner die Verwendung der in Schritt (c) und/oder Schritt (ca) erhältlichen D-Nucleinsäuren als Matrize zur Herstellung einer L-Nucleinsäure mit identischer Nucleinsäuresequenz.

Weiterhin umfaßt die Erfindung Arzneimittel, enthaltend eine nach den vorstehend beschriebenen Verfahren erhältliche L-Nucleinsäure oder eine der vorstehend beschriebenen L-Nucleinsäuren, gegebenenfalls in Kombination mit einer kovalenten Modifikation und/oder einem pharmazeutisch verträglichen Träger.

Die erfindungsgemäßen Arzneimittel können in vielfältiger Weise eingesetzt werden. Der Wirkungsbereich des Arzneimittels hängt aber selbstverständlich von der Spezifität der L-Nucleinsäure ab. Die erfindungsgemäßen Arzneimittel können beispielsweise in der Behandlung von Krebs, viralen und bakteriellen Infektionen, und Bluthochdruck eingesetzt werden. Die jeweilige Applikationsform, die Dosis und die Dauer der Behandlung wird vom behandelnden Arzt von Fall zu Fall festgesetzt, wobei die Schwere der Krankheit, das Alter und der allgemeine Zustand des Patienten einige der Parameter sind, die vom behandelnden Arzt berücksichtigt werden. Sofern erforderlich, wird die erfindungsgemäße L-Nucleinsäure zusammen mit einem pharmazeutisch verträglichen Träger verabreicht. Die Wahl dieser Zusatzstoffe hängt u.a. von der Applikationsart ab. Der Fachmann weiß aber aus dem Stand der Technik. welche Träger er dem jeweiligen Arzneimittel zuzusetzen hat.

Schließlich betrifft die Erfindung einen Kit und ein diagnostisches Mittel, enthaltend eine nach den vorstehend beschriebenen Verfahren erhaltene L-Nucleinsäure oder eine vorstehend beschriebene L-Nucleinsäure. Der erfindungsgemäße Kit kann für diagnostische und analytische Zwecke verwendet werden. Da die erfindungsgemäßen L-Nucleinsäuren, wie vorstehend beschrieben. in ähnlicher Weise wie Antikörper eingesetzt werden können, bietet sich dem Fachmann als Einsatzgebiet für den erfindungsgemäßen Kit das gesamte Spektrum der diagnostischen Möglichkeiten von polyklonalen oder monoclonalen Antikörpern. Beispielsweise kann die L-Nucleinsäure des erfindungsgemäßen Kits mit einem Marker versehen und zum *in vitro*-Nachweis des Zielmoleküls verwendet werden.

In Verbindung mit z. B. Oberflächen-Plasmon-Resonanz-Sensoren, evaneszenten Feld-Sensoren oder Gitterkopplem ist es ebenfalls möglich. L-Nucleinsäuren als Biosensoren einzusetzen. Somit ist ein weiterer Gegenstand der Erfindung die Verwendung der mit dem erfindunggemäßen Verfahren erhältlichen L-Nucleinsäuren oder der erfindungsgemäßen L-Nucleinsäuren als Biosensoren: Es ist ebenfalls denkbar, daß die erfindungsgemäßen L-Nucleinsäuren als Herbizide, Zusatzstoffe für Nahrungsmittel, für analytische Verfahren, z.B. zum Nachweis von Geruchs- und/oder Geschmacksstoffen oder für kosmetische Anwendungen wie Anti-Falten- und Sonnencremes benutzt werden können.

Diese und andere Ausführungsformen sind dem Fachmann offenbart und offensichtlich und umfaßt durch die Beschreibung und die Beispiele der vorliegenden Erfindung. Zum Beispiel kann weiterführende Literatur zu einer der oben angeführten Methoden, Mittel und Verwendungen, die im Sinne der vorliegenden Erfindung angewendet werden können, dem Stand der Technik entnommen werden, z. B. aus öffentlichen Bibliotheken unter z. B. der Benutzung von elektronischen Hilfsmitteln. Zu diesem Zweck bieten sich unter anderem öffentliche Datenbanken wie die "Medline", die über Internet zur Verfügung stehen, z. B. unter der Adresse http://www.ncbi-nlm.nih.gov/PubMed/medline.html. Weitere Datenbanken und Adressen sind dem Fachmann geläufig und können aus dem Internet entnommen werden, z. B. unter der Adresse http://www.lycos.com. Eine Übersicht über Quellen und Informationen zu Patenten bzw. Patentanmeldungen in der Biotechnologie ist in Berks, TIBTECH 12 (1994), 352-364 gegeben.

Die Figuren zeigen:
- Fig. 1:: Design von hochaffinen RNAs. In Schritt (a) wird zunächst eine heterogene DNA-Matrize hergestellt. Die Synthese dieser Matrize kann chemisch erfolgen. Die Matrize kann jedoch beispielsweise auch aus einem Spaltprodukt natürlich vorkommender DNA und daran über eine Ligierungsreaktion angeheftete Primer für die PCR bestehen. Im nachfolgenden Schritt (b) wird die Matrize durch PCR amplifiziert. Die erhaltenen DNAs werden in Schritt (c) transkribiert und anschließend mit dem Zielmolekül in Kontakt gebracht. (Hoch)affine RNAs binden an das Zielmolekül. Nach Abtrennung der nicht gebundenen Moleküle werden die (hoch)affinen RNAs in vitro mit reverser Transkriptase in cDNA umgeschrieben. In Schritt (f) schließlich wird der komplementäre cDNA-Strang synthetisiert, wodurch man wieder ein doppeisträngiges cDNA-Molekül erhält. Dieses Molekül kann anschließend erneut amplifiziert und auf spezifische Bindung hin selektiert werden.
- Fig. 2:: Spiegelselektion: In Schritt (a) wird eine Population heterogener D-DNAs oder D-RNAs bereitgestellt. Diese werden in Schritt (b) mit der optischen Antipode des Zielmoleküls in Kontakt gebracht. D-Nucleinsäuren, die mit der optischen Antipode des Zielmoleküls in Wechselwirkung treten, werden von den anderen D-Nucleinsäuren abgetrennt (Selektion). Sofern eine genügend große Anzahl von relativ einheitlicher Zusammensetzung mit der optischen Antipode des Zielmoleküls in Wechselwirkung getreten sind, können diese gemäß der von Blackwell et al. (a.a.O) beschriebenen Strategie sequenziert werden. Mikrosequenzierverfahren sind beispielsweise von Davis et al. (Genet. Anal. Tech. Appl. 8 (1991), 1-7), sowie Harding und Keller (Trends Biotechnol. 10 (1992), 55-57) beschrieben worden. Anhand der erhaltenen Sequenzinformation kann dann die entsprechende L-DNA oder L-RNA synthetisiert werden (Schritt d). Diese L-DNA oder L-RNA wird dann spezifisch mit dem Zielmolekül in Wechselwirkung treten (Schritt e).
- Fig. 3:: Spiegelselektion bzw. -Evolution. Die Verfahrensschritte entsprechen den in Fig. 2 dargestellten, mit der Ausnahme, daß nach dem Selektionsschritt (b) das selektionierte Material amplifiziert wird. Sofern beim amplifizierten Material Fehler bei der *de novo* cDNA- oder RNA-Synthese auftreten, das amplifizierte Material hinsichtlich der Sequenzinformation also heterogener ist als die Ausgangspopulation, beinhaltet der Amplifikations- auch einen Variationsschritt. Damit sind sämtliche, die biologische Evolution treibenden Kräfte in dem System *in vitro* vereint.

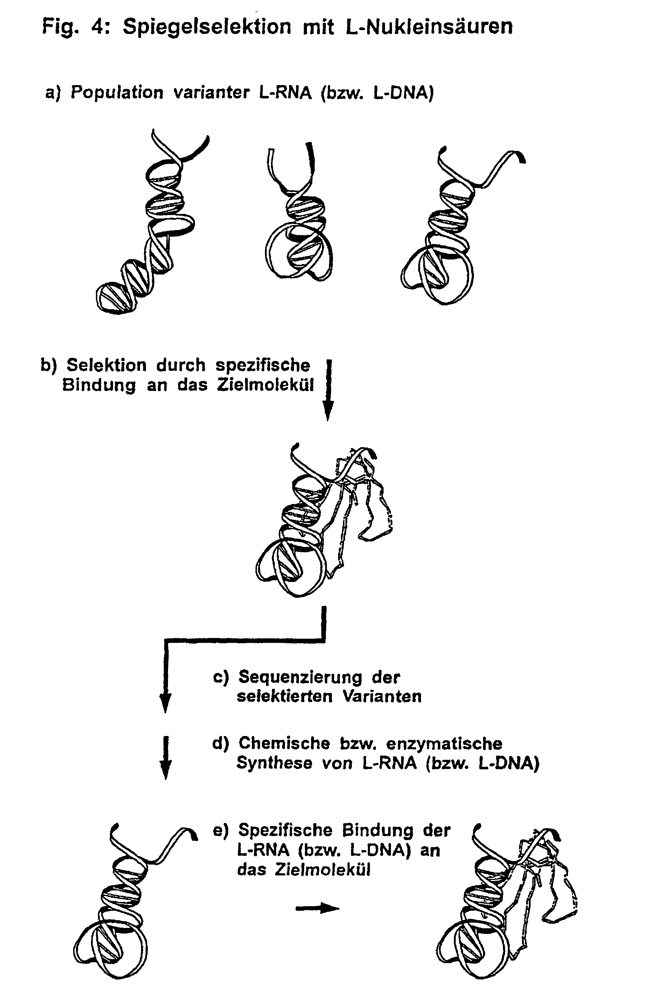

- Fig. 4:: Spiegelselektion mit L-Nukleinsäuren. Spiegelselektion mit L-Nukleinsäuren: In Schritt (a) wird eine Population heterogener L-DNAs oder L-RNAs bereitgestellt. Diese werden in Schritt (b) mit dem Zielmolekül in Kontakt gebracht. L-Nukleinsäuren, die mit dem Zielmolekül in Wechselwirkung treten, werden von den anderen L-Nukleinsäuren abgetrennt (Selektion). Sofern eine eine genügend große Anzahl von L-Nucleinsäuren von relativ einheitlicher Zusammensetzung mit der optischen Antipode des Zielmoleküls in Wechselwirkung getreten sind, können diese gemäß der von Blackwell et al. (a.a.O) beschriebenen Strategie sequenziert werden, wobei entsprechende L-Proteine hier durch D-Proteine ersetzt werden. Mikrosequenzierverfahren sind beispielsweise von Davis et al. (a.a.O), sowie Harding und Keller (a.a.O) beschrieben worden. Anhand der erhaltenen Sequenzinformation kann dann die entsprechende L-DNA oder L-RNA synthetisiert werden (Schritt d). Diese L-DNA oder L-RNA wird dann spezifisch mit dem Zielmolekül in Wechselwirkung treten (Schritt e).
- Fig. 5:: Spiegelselektion bzw. -evolution. Die Verfahrensschritte entsprechen den in Fig. 3 dargestellten, mit der Änderung, daß eine Population heterogener L-Nukleinsäuren bereitgestellt wird und die dort verwendeten L-Proteine durch D-Proteine ersetzt werden. Die Sequenzinformationen können nach iterativer *in vitro*-Vereinzelung bestimmt werden (siehe Beispiel 4).
- Fig. 6:: Darstellung von L-Ribophosphoramidit-Synthonen.
- Fig. 7:: Synthese von trägergebundenen L-Ribonucleosiden (geschützt).
- Fig. 8:: Festphasensynthese von L-RNA.
- Fig. 9:: Darstellung von L-Desoxyribophosphoramidit-Synthonen.
- Fig. 10:: Synthese von trägergebundenen L-Desoxyribonucleosiden (geschützt).
- Fig. 11:: Festphasensynthese von L-DNA.

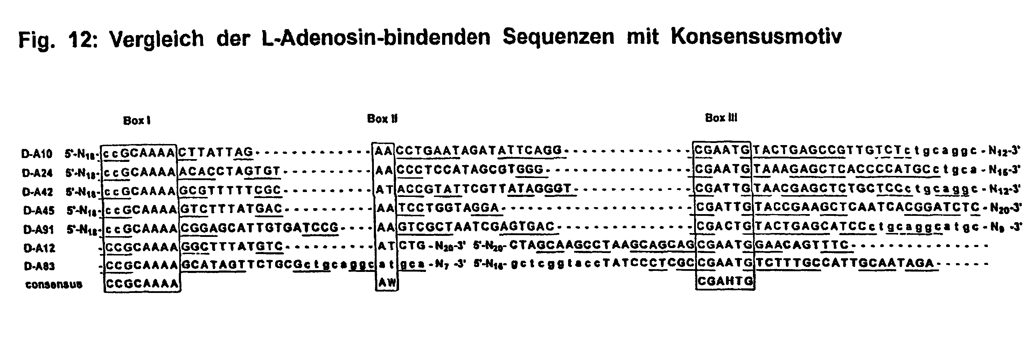

- Fig. 12:: Vergleich der L-Adenosin-bindenden Sequenzen mit Konsensusmotiv. Die umrahmten Nucleotide zeigen Bereiche mit konservierter Sequenz. W steht für A oder T und H steht für A, C oder T. Das Motiv der Box II konnte erst durch den Vergleich gemeinsamer Sekundärstrukturen zugeordnet werden. Nucleotide der Region mit ursprünglicher Zufallssequenz werden duch große Buchstaben repräsentiert, während Nucleotide der Primer-Regionen durch kleine Buchstaben markiert sind. Unterstrichene Positionen nehmen im Sekundärstruktur-Modell an Basenpaarungen teil. Um die Sequenzen von D-A12 und D-A83 in das Konsensusdiagramm einzufügen, wurden die 5'-Termini in die Mitte des Schemas gelegt.
- Fig. 13:: Sekundärstrukturmodell von D-A42d. Konservierte Nucleotide sind umkreist.
- Fig. 14:: Bindung von D-A42d an L-Adenosin ( ) und L-A42d an D-Adenosin (○).
- Fig. 15:: Enzymatischer Verdau von D-A42d. In den Spuren a-h wurden 5'-markierte Oligoribonucleotide, in den Spuren i-p wurden 3'-markierte Oligoribonucleotide untersucht. Spur a, g, i und o: Alkalileiter; Spur b und n: RNase T₁ (denaturierende Bedingungen); Spur c und m: RNase T₁; Spur d und l: RNase V₁; Spur e und k: RNase T₂; Spur f und j: Nuclease S₁; Spur h und p: ungespalten.
- Fig. 16:: CD-Spektren von D-A42d und L-A42d.
- Fig. 17:: Kompetitive Bindungskurven von L-A42d. Gezeigt sind die Kompetitionen mit D-Adenosin (○), D-Guanosin (●), D-Uridin (□), D-Cytidin (■), und L-Adenosin (Δ). Jedes Experiment wurde zweifach durchgeführt.
- Fig. 18:: Kompetitive Bindungskonstanten von L-A42d. Gezeigt sind die Dissoziationskonstanten (K_{d}c) von Nucleosidanaloga und relative Bindungsaffinitäten (K_{d}c/K_{d}D-A) von Kompetitor zur D-Adenosin Kompetition (K_{d}D-A).
- Fig. 19:: Stabilität der Oligonucleotid-Liganden in Humanserum. (A) D-Oligoribonucleotid D-A42d und (B) L-Oligoribonucleotid L-A42d. Aliquots wurden zu den indizierten Zeiten entnommen. L markiert den Größenstandard (10 bp Leiter). Die Ergebnisse wurden in einem unabhängigen Experiment reproduziert.
- Fig. 20:: Vergleich der D-Arginin-bindenden Sequenzen mit Konsensusmotiv. Die Analyse lieferte zwei Klassen von RNA-Molekülen (bezeichnet als D-RA und D-RB), die jeweils zwei konservierte Sequenzmotive (bezeichnet als seq 1 und seq 2) enthielten. Nucleotide der Region mit ursprünglicher Zufallssequenz werden duch große Buchstaben repräsentiert, während Nucleotide der Primer-Regionen durch kleine Buchstaben markiert sind. Unterstrichene Positionen nehmen im Sekundärstruktur-Modell an Basenpaarungen teil.
- Fig. 21:: Sekundärstrukturmodell der Konsensusmotive von D-RA und D-RB.

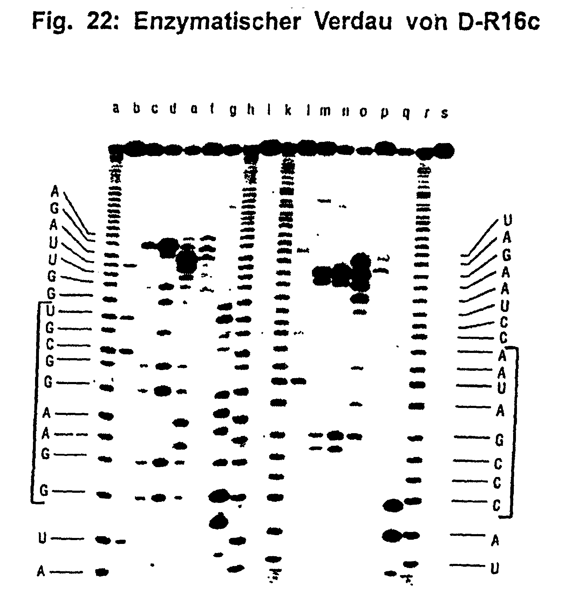

- Fig. 22:: Enzymatischer Verdau von D-R16c. Die Figur zeigt ein Autoradiogramm von enzymatischen Verdauungsprodukten, die auf einem denaturierenden Polyacrylamidgel analysiert wurden. Die Nucleotide in den konservierten Motiven sind durch Klammern markiert. In den Spuren a-i wurden 5'-markierte Oligoribonucleotide, in den Spuren k-s wurden 3'-markierte Oligoribonucleotide untersucht. Spur a, h, k und r: Alkalileiter; Spur d-g und n-q: native Bedingungen. Spur d und n: RNase T₁. Spur e und o: RNase T₂. Spur fand p: RNase S₁. Spur g and q: RNase V₁. Spur b, c, 1, and m: denaturierende Bedingungen. Spur b and 1: B. cereus. Spur c and m: RNase T₁.
- Fig. 23:: Sekundärstrukturmodell von D-R16c. Die Pfeile markieren die Schnittstellen von RNase T₁ (◆), RNase T₂ (■), RNAse S₁ ( ), and RNase V₁ (●). Gefüllte Symbole indizieren starke Spaltung, ungefüllte Symbole zeigen schwache Spaltung an.
- Fig. 24:: Bindung von D-R16c an D-Arginin (■) und L-Arginin (□).
- Fig. 25:: CD-Spektren von D-R16c und L-R16c.
- Fig. 26:: Bindung von L-R16c an L-Arginin (●) und D-Arginin (○).
- Fig. 27:: Kompetitive Bindingsanalyse von L-R16c mit L-Arginin ( ) und Tat-Peptid (Δ).
- Fig. 28:: Iterative in vitro-Vereinzelung und Sequezierung. Zunächst wird die Population heterogener RNA-Moleküle durch Reverse Transkriptase in cDNA-Moleküle übersetzt. Nach Komplementärstrangsynthese werden die DNA-Moleküle durch PCR amplifiziert. Durch Verdünnung bzw. Aliquotierung kann die Heterogenität der Population verringert werden. Nach anschließender PCR-Vermehrung kann das Verfahren zyklisch durchlaufen werden, bis die DNA-Moleküle vereinzelt sind. Die Sequenz läßt sich dann durch Didesoxysequenzierung ermitteln.
- Fig. 29:: Nach iterativer in vitro-Vereinzelung bestimmte Sequenzen.

Die Beispiele erläutern die Erfindung.

### Beispiel 1

### Synthese von L-Oligoribonucleotiden

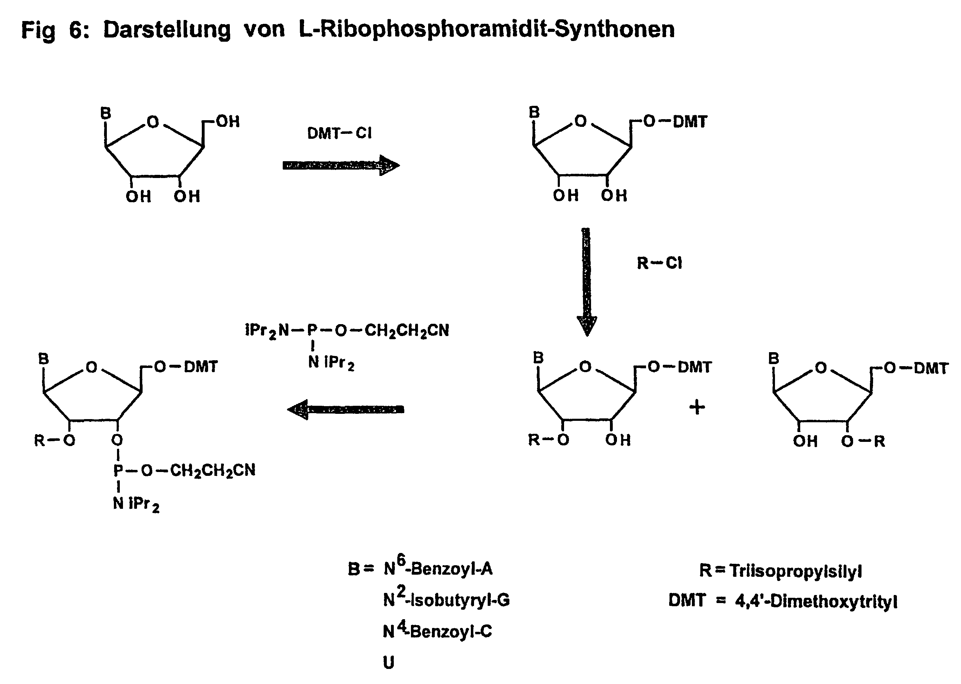

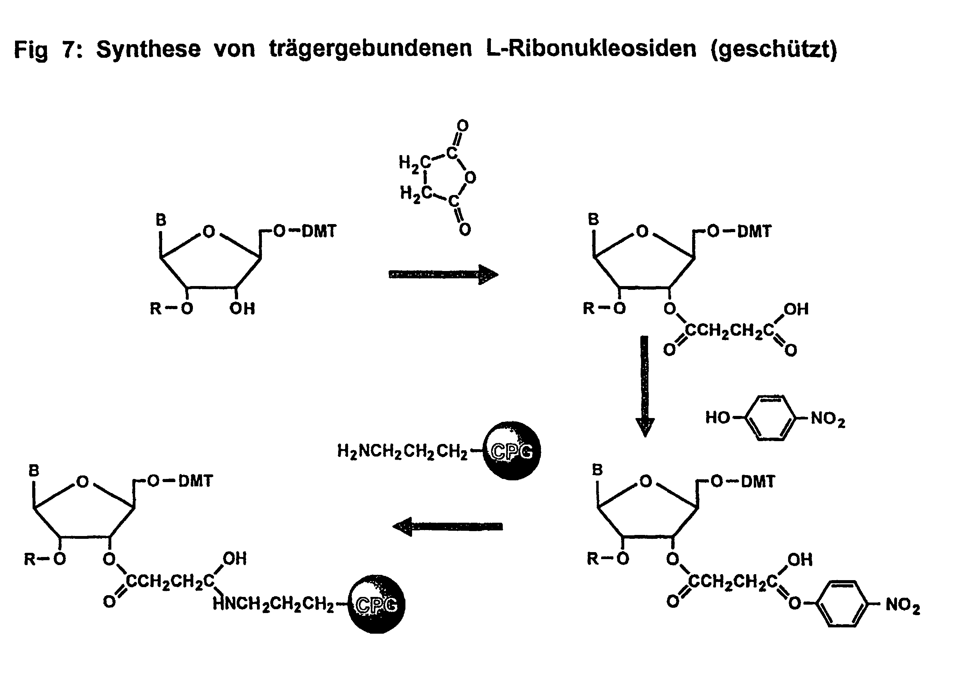

L-Adenosin wurde augehend von L-Arabinose nach Hol 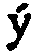 und 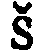 orm (Collect. Czech. Chem. Commun. 34 (1969) 3383-3401) über Benzyl-β-L-arabinopyranosid und Umwandlung in 2-*O*-Tosyl-5-*O*-trityl-L-arabinose präpariert. Zur Synthese von L-Uridin wurde zunächst aus L-Arabinose das 2,2'-*O*-Anhydro-L-uridine nach Hol 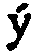 (Collect. Czech. Chem. Commun. 37 (1972), 4072-4087) dargestellt. Nach Hol 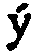 (Collect. Czech. Chem. Commun. 38 (1973), 423-427) wurde dann zum 3',5'-Di-O-benzoyl-Derivat benzoyliert und nach Reaktion mit Bortrifluorid-etherat das benzoylierte L-Uridin alkalisch entblockt. L-Cytidin und L-Guanosin wurden, wie von Vorbrüggen et al. (Chem. Ber. 114 (1981), 1234-1255) für D-Nucleoside beschrieben, aus den silylierten Heterozyklen and der peracylierten Pentose gewonnen, wobei analog zu Zou und Robins (Can. J. Chem. *65* (1987), 1436-1437) das 2-*N*-acetyl-*O*-diphenylcarbamoylguanin für die Synthese von L-Guanosin eingesetzt wurde. Zur Darstellung der peracylierten Pentose wurde zunächst nach Abe et al. (Chem. Pharm. Bull. 28 (1980), 1324-1326) die L-Ribose durch Epimerisierung aus der L-Arabinose gewonnen und dann in einer dreistufigen Synthese, wie von Recondo und Rinderknecht (Helv. Chim. Acta 42 (1959), 1171-1173) für das D-Isomere beschrieben, zum 1-*O*-Acetyl-2,3,5-tri-*O*-benzoyl-ß-L-ribofuranosid derivatisiert. Zur Präparation der Phosphoramidite für die Festphasensynthese (Fig. 6) wurden, wie für D-Nucleoside beschrieben, die exozyklischen Aminogruppen der Nucleoside durch Benzoylierung von Adenosin und Cytidin nach Ti et al. (J. Am. Chem. Soc. 104 (1982), 1316-1319) und Isobutyrylierung nach Flockerzi et al. (Liebigs Ann. Chem (1981), 1568-1585) geschützt. Die Basen-geschützten Nucleoside und Uridin wurden in Analogie zu Usman et al. (J. Am. Chem. Soc. 109 (1987), 7845-7854) in ihre 5'-*O*-Dimethoxytrityl-2'-O-triisopropylsilyl Derivativen überführt. Die Derivate wurden dann in Analogie zu Milecki et al. (Nucleosides Nucleotides 8 (1989), 463-474) zu ihren jeweiligen 3'-*O*-(β-Cyanoethyl-N,N-diisopropyl) phosphoramiditen umgesetzt. Trägergebundene geschützte Nucleoside wurden, wie von Usman et al. (J. Am. Chem. Soc. 109 (1987), 7845-7854) für die D-Isomere beschrieben, hergestellt (Fig. 7).

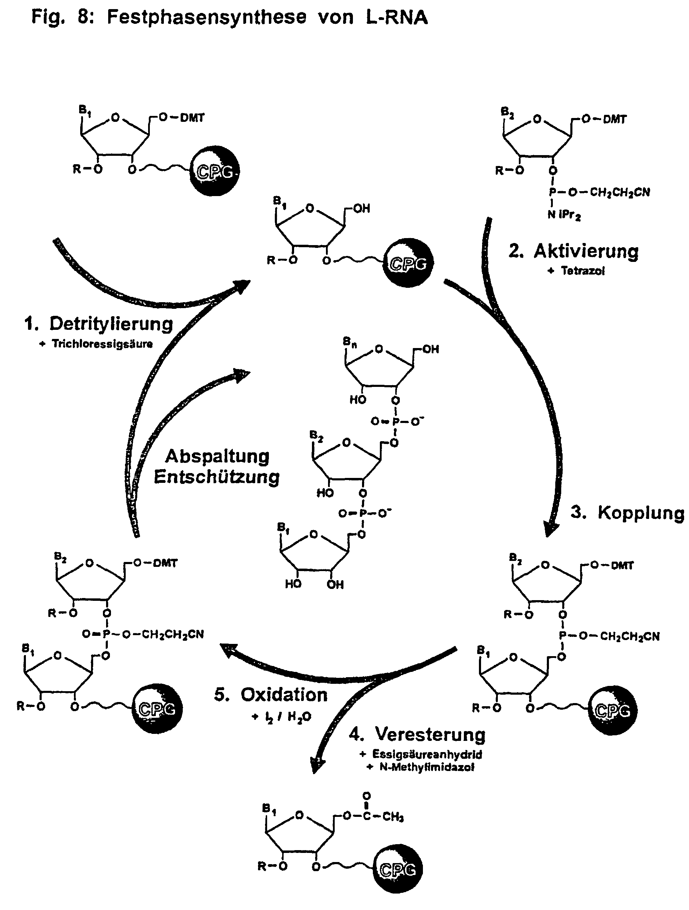

Für die chemische Festphasensynthese der L-Oligoribonucleotide wurden Geräte der Firma Applied Biosystems eingesetzt (Modell 391 PCR-MATE™ EP und Modell 394). Die Synthesen wurden im 0,2 µmol Maßstab mit dem DNA-Standardzyklus durchgeführt (Fig. 8), wobei der Kopplungsschritt auf 15 Minuten ausgedehnt wurde.

Nach der Synthese wurde das trägergebundene, geschützte L-Oligoribonucleotid für 24 Stunden in 1 ml eines 3:1-Gemisches aus 32%igem Ammoniak und Ethanol (v/v) bei 55 °C inkubiert, um das Oligonucleotid vom Träger zu trennen und die Schutzgruppen abzuspalten. Die Lösung wurde abgenommen und das Trägermaterial mit 400 µl eines 1:1-Gemisches aus Ethanol und Wasser (v/v) nachgewaschen. Den vereinigten Überständen wurde ein 5 µl Aliquot entnommen und die UV-Absorption bei 260 nm bestimmt. Anschließend wurde die Probe bis zur Trockne eingeengt. Zum Abspalten der 2'-Hydroxyl-Schutzgruppe wurde das Oligoribonucleotid in (10 x A₂₆₀) µl Tetrabutylammoniumfluorid (1.1 M in Tetrahydrofuran) und (1 x A₂₆₀) µl Ethanol-Wasser (1:1, v/v) gelöst und für 72 h bei RT inkubiert.

Das überschüssiges Tetrabutylammoniumfluorid wurde mit einer tip 500-Säule (QIAGEN) entfernt. Dazu wurde die Probe mit 0.1 M Triethylammoniumacetat (TEAAc), pH 7.0, auf ein Gesamtvolumen von 10 ml verdünnt und auf die mit 0.1 M TEAAc, pH 7.0, äquilibrierte Säule gegeben. Die Säule wurde zweimal mit je 30 ml 0.1 M TEAAc, pH 7.0, gewaschen und die Probe dann mit 10 ml 2 M TEAAc, pH 7.0, eluiert. Das Eluat wurde zur Trockne eingeengt und über denaturierende Polyacrylamidgelelektrophorese gereinigt. Die Produktbande wurde durch UV-Shadowing detektiert und ausgeschnitten. Das L-Oligoribonucleotid wurde aus dem Gelstück für 12 Stunden mit H₂O eluiert und das Eluat über eine NAP™10 Säule (Pharmacia) entsalzt.

### Synthese von L-Oligodesoxyribonucleotiden

L-2'-Desoxyadenosin und L-2'-Desoxyguanosin wurden ausgehend von L-Adenosin bzw. L-Guanosin durch Reduktion, wie von Robins et al. (J. Am. Chem. Soc. 105 (1983), 4059-4065) für D-Nucleoside beschrieben wurde, dargestellt. L-2'-Desoxythymidin und L-2'-Desoxycytidin wurden nach Hol 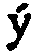 (Collect. Czech. Chem. Commun. 37 (1972), 4072-4087) in mehreren Stufen präpariert: Das Zwischenprodukt 3',5'-Di-O-Benzoyl-2,2'-O-anhydro-L-uridin aus der L-Uridin-Synthese wurde in das 2'-Chloro-2'-desoxy-Derivat überführt, das nach Reduktion und Deblockierung L-2'-Desoxyuridin lieferte. Die Umsetzung mit Formaldehyd in Kalilauge und anschließende katalytische Reduktion lieferte L-2'-Desoxythymidin. L-2'-Desoxycytidin wurde aus 3',5'-Di-O-Benzoyl-2'desoxyuridin nach Überführung in das 4-Thio-Derivat durch Umsetzung mit methanolischem Ammoniak unter Druck erhalten.

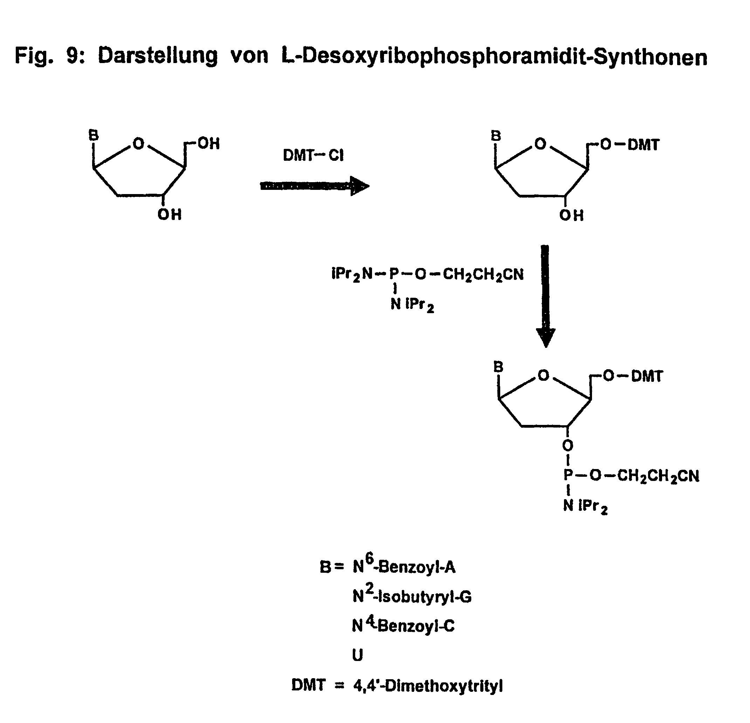

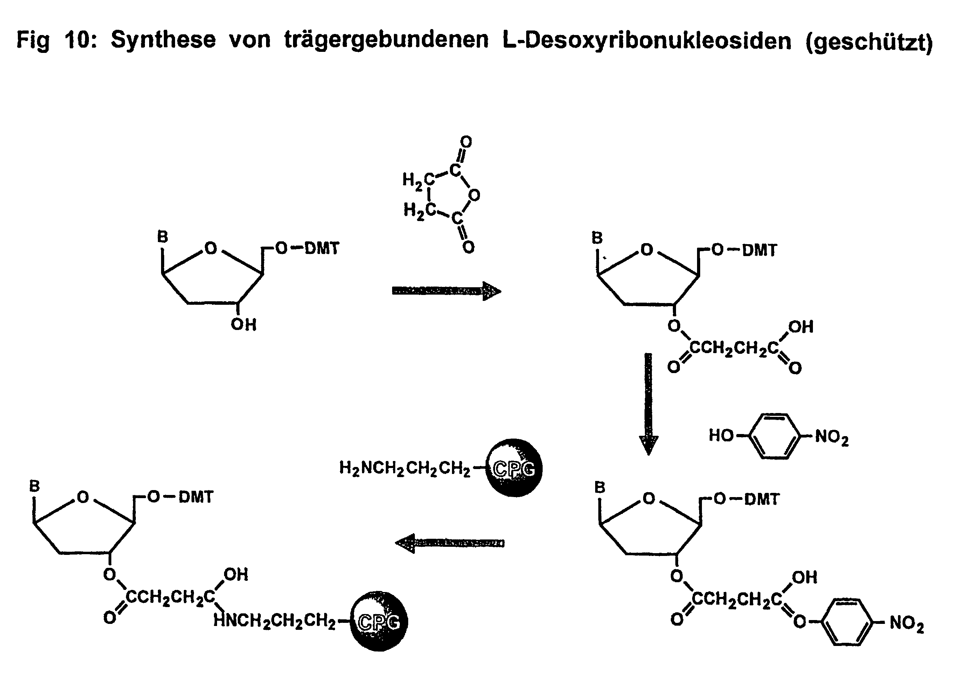

Die Präparation der Phosphoramidite wurde, wie für D-Desoxynucleoside beschrieben, ausgeführt (Fig. 9). Exocyclischen Aminogruppen wurden nach Ti et al., a.a.O. beim L-2'-Desoxyadenosin und L-2'-Desoxycytidin benzoyliert und beim 2'-Desoxyguanosin isobutyryliert. Die 5'-Hydroxylgruppe der L-2'-Desoxynucleoside wurde nach Schaller et al. (J. Am. Chem. Soc. 85 (1963), 3821-3827) mit 4,4'-Dimethoxytritylchlorid zu den entsprechenden Tritylethern umgesetzt. Die L-2'-Desoxynucleosid-Phosphoramidite wurde nach Milecki et al., a.a.O. durch Umsetzung der geschützen L-2'-Desoxynucleoside mit β-Cyanoethyl-N,N,N',N'-Tetraisopropylphosphordiamidit präpariert. Trägergebundene, geschützte L-2'-Desoxynucleoside wurden analog zu den D-Isomeren nach Atkinson und Smith (in Gait (Ed.), Oligonucleotide Synthesis, 1984, IRL Press, Oxford, p. 35-81) dargestellt (Fig. 10).

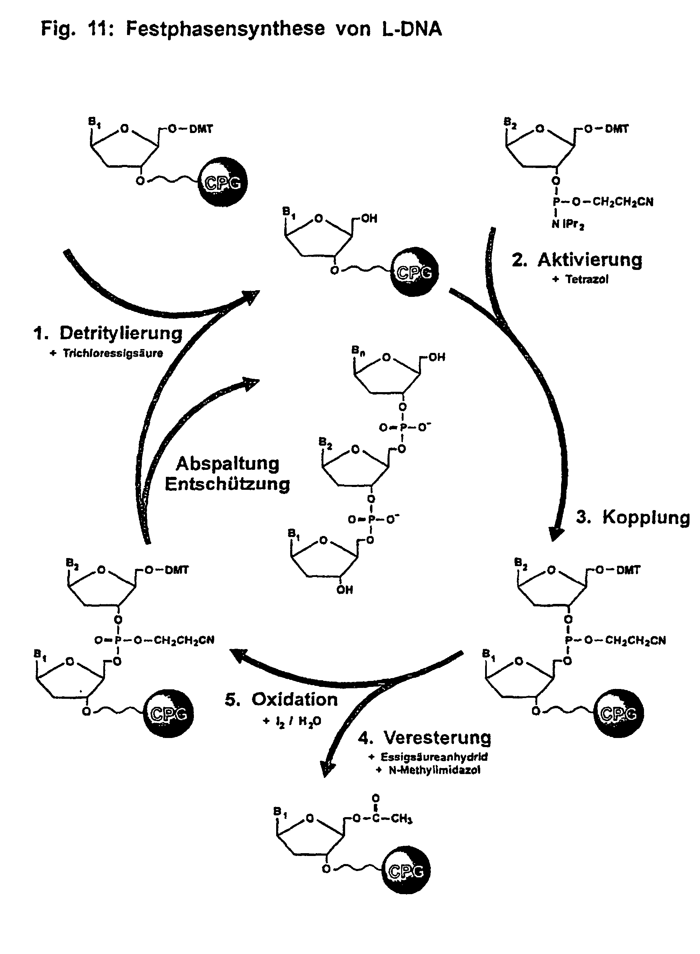

Die Festphasensynthese der L-Oligodesoxyribonucleotide wurde an Geräten der Firma Applied Biosystems (Modell 391 PCR-MATE™ EP und Modell 394) im 0,2 µmol DNA-Standardzyklus durchgeführt (Fig. 11).

Nach der Synthese wurde das trägergebundene, geschützte L-Oligodesoxyribonucleotid für 24 Stunden in 1 ml 32%igem Ammoniak bei 55 °C inkubiert, um das Oligonucleotid vom Träger zu trennen und die Phosphat- bzw. Basenschutzgruppen abzuspalten. Die Lösung wurde abgenommen und zur Trockne eingeengt. Das aufgenommene L-Oligodesoxyribonucleotid wurde über denaturierende Polyacrylamidgelelektrophorese gereinigt. Die Produktbande wurde durch UV-Shadowing detektiert und ausgeschnitten.

Das L-Oligodesoxyribonucleotid wurde aus dem Gelstück für 12 Stunden mit H₂O eluiert und das Eluat über eine NAP™10 Säule (Pharmacia) entsalzt.

### Beispiel 2

### Verfahren zur Identifikation von D-Adenosin-spezifischen L-Oligoribonucleotiden

### Kopplung von L-Adenosin

Zur Selektion der affinen Oligonucleotid-Liganden durch Affinitätschromatographie wurde L-Adenosin an Sepharose immobilisiert. Das L-Nucleosid wurde, wie in Beispiel 1 beschrieben, synthetisiert und in Analogie zu Jones und Robins (J. Am. Chem. Soc. 85 (1963), 193-201) mit Jodessigsäure zum 1-Carboxymethyl-L-adenosin alkyliert. Nach alkalischer Umlagerung in 0,25 M NaOH für 2 Stunden bei 95° C wurde das entstehende N⁶-Carboxymethyl-L-adenosin nach Lindberg und Mosbach (Eur. J. Biochem. 53 (1975), 481-486) mit 1,6-Diaminohexan in der Gegenwart von N-(3-Dimethylaminopropyl)-N'-ethylcarbodiimid zum N⁶-[(6-Aminohexyl)-carbamoylmethyl]-L-adenosin kondensiert. Das L-Adenosin-Derivat wurde zu einer Endkonzentration von 3,5 mM an CNBr-aktivierte Sepharose 4B (Pharmacia) gekoppelt.

### Identifizierung eines Adenosin-Bindungsmotivs

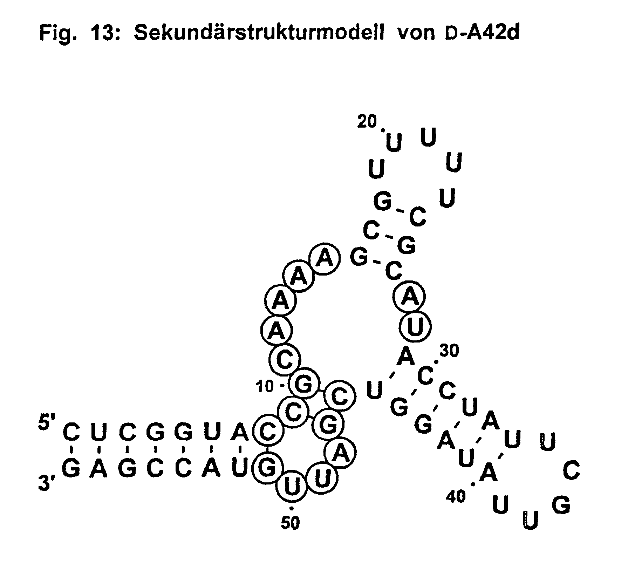

Eine kombinatorische D-RNA Bibliothek mit etwa 10¹⁵ Zufallssequenzen wurde durch *in vitro*-Transkription mit T7 -RNA-Polymerase aus einem Gemisch von DNA Molekülen gewonnen, die eine 60-Nucleotide lange Region mit Zufallssequenzen enthielten. Begrenzt wurde die Region durch 20 Nucleotide lange DNA-Bereiche mit vorgegebener Sequenz: (5'-CCA'AGC'TTG'CAT'GCC'TGC'AGN60'GGT'ACC'GAG'CTC'GAA'TTC'CC-3'). Um die Anreicherung Säulenmaterial-affiner Liganden zu vermeiden, wurde die RNA in allen Selektionsrunden zunächst über Vorsäulen mit unbeladenem Säulenmaterial gegeben. In der ersten Selektionsrunde betrug das Säulenbettvolumen der Vorsäule 150 µl, das der Hauptsäule 400 µl. Etwa 4 nmol Pool-RNA (1,3 x 10¹⁵ verschiedene Moleküle) wurden in Bindungspuffer (250 mM NaCl, 40 mM Tris/HCl pH 7.6, 5 mM MgCl₂ und 0.5 mM EDTA) auf die Säulen aufgetragen. Von der Affinitätssäule konnten im ersten Selektionszyklus 0.06-0.07% der eingesetzten RNA mit 15 mM L-Adenosin in Bindungspuffer spezifisch eluiert werden. Für alle weiteren Selektionsrunden wurden etwa 1 nmol RNA aufgetragen und 100 µl-Vorsäulen bzw. 250 µl-Hauptsäulen verwendet. Im sechsten Selektionszyklus konnten 38% der gebundenen RNA-Moleküle spezifisch mit L-Adenosin eluiert werden. Dieser Zyklus wurde wiederholt, um die Stereospezifität der Liganden zu erhöhen. Vor der Elution mit L-Adenosin wurde das Affinitätsmaterial mit D-Adenosin gewaschen, wobei der Anteil der mit L-Adenosin eluierten RNAs auf 18% reduziert wurde. Nach weiteren vier Selektionszyklen war die Fraktion spezifisch gebundener RNA-Liganden wieder auf 40% angestiegen. Die PCR-Produkte dieser Runde wurden mit den Restriktionsendonucleasen *Eco*RI and *Pst*I verdaut, in das Vektorplasmid pT7/T3 (Gibco BRL) kloniert und nach der Methode von Sanger sequenziert. Die Sequenzanalyse der selektierten RNA-Moleküle ergab ein Konsensusmotiv, das in 20% der sequenzierten Klone gefunden wurde (Fig. 12). Für die Sequenzen mit Konsensusmotiv konnte ein gemeinsames Sekundärstrukturmodell vorgeschlagen werden (Fig. 13).

### Charakterisierung der L-Adenosin-spezifischen RNA-Moleküle

Die individuellen Dissoziationskonstanten (*K*_{d}) wurden durch Gleichgewichtsdialyse in Micro-Dialysekammern bestimmt. Die beiden Kompartimente wurden durch eine Spectra/Por® (Spectrum) Celluloseester-Dialysemembran (Molekulargewichts-Ausschlußgrenze von 2000) getrennt. Die RNA-Konzentrationen wurden im Spektralphotometer bei 260 nm bestimmt. Die individuellen Extinktionskoeffizienten der RNA-Moleküle wurden nach A.J. Zaug et al. (Biochemistry 27 (1988), 8924-8931) durch vollständige alkalische Hydrolyse ermittelt. Zur Messung der Dissoziationskonstante wurde die RNA in Bindungspuffer bei 90 °C für 10 Minuten denaturiert, innerhalb von 10 Minuten auf Raumtemperatur abgekühlt, und dann mit Konzentrationen von 0.1 bis 50 µM gegen eine Lösung von 10 nM D-[2,8-³H]Adenosin (Moravek Biochemicals, Inc.) bzw. L-[2,8(n)-³H]Adenosin (tritiiert von Amersham) in Bindungspuffer äquilibriert. Jedes Kompartiment wurde mit 40 µl Lösung gefüllt. Nach Inkubation für 24 h bei 18 °C wurden 35 µl Aliquote entnommen und die Radioaktivität der Proben im Szintillationszähler vermessen. Die Differenz der Radioaktivität in den beiden Kompartimenten relativ zur Radioaktivität in dem RNA-enthaltenden Kompartiment wurde als prozentuale Adenosin-Bindung an RNA genommen. Die Daten wurden durch nicht-lineare Regressionsanalyse nach Connors (Binding constants, 1987, John Wiley & Sons, New York) an eine Standard-Bindungsgleichung für 1:1 Stöchiometrie angepaßt.

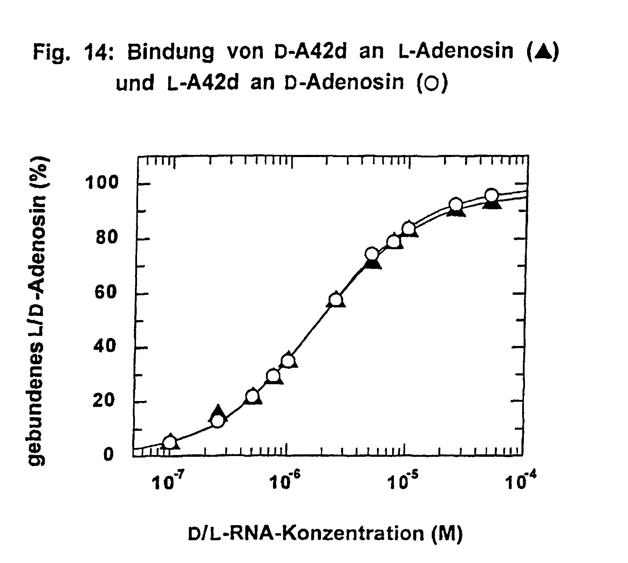

Die T7-Transkripte der Klone mit dem Konsensusmotiv zeigten Dissoziationskonstanten zwischen 1.1 and 10 µM für die Bindung von L-Adenosin. Von dem besten Liganden D-A42 (*K*_{d} von 1.1 ± 0.1 µM) wurde verkürzte Versionen untersucht. Das 58-mer D-A42d (Fig. 13) zeigte eine Dissoziationskonstante von 1.7 ± 0.1 µM (Fig. 14). Neben Verkürzungen am 5'- und 3'-Enden fehlen D-A42d zwei G Nucleotide zwischen Position 31/32 und 41/42. Zusätzlich wurde in Position 54 A durch C ausgetauscht.

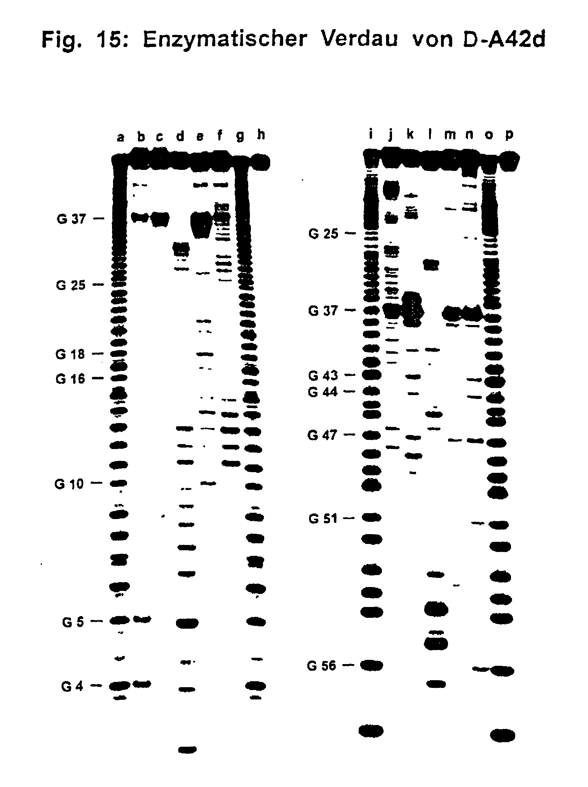

Das Sekundärstrukturmodell von D-A42d steht in Übereinstimmung mit den durch Nuclease-Verdau gewonnenen Daten (Fig. 15). Für den nativen Verdau wurden die 5'und 3'-gelabelten Oligoribonucleotide bei 90 °C denaturiert und in Bindungspuffer hybridisiert. Für jede Spaltungsreaktion wurden 100,000 cpm (≈ 30 fmol) markiertes Oligoribonucleotid eingesetzt. Die Spaltungen wurden, wie von G. Knapp (Methods Enzymol. 180 (1989), 192-212) beschrieben, durchgeführt. Die Spaltungsprodukte wurden auf denaturierenden 25% Polyacrylamidgelen getrennt. Das einzige G in der vorhergesagten einzelsträngigen Region bei Position 37 wurde durch RNase T1 deutlich gespalten. Die Spaltungen von RNase T₂ bei Positionen G47 und A48 und der Einzelstrang-spezifischen Nuclease S₁ bei Positionen G10, C11, A12, A13 und A27 bestätigen die konservierten internen Loops zwischen G10 / G16 und G47 / G51, sowie die Loop-Region zwischen U34 / A40. Die Spaltungen mit der Doppelstrang-spezifischen RNase V₁ bei Positionen U2-A7, G10, C30-U32, U45 und U52-G56 stehen in Übereinstimmung mit dem Sekundärstrukturmodell. Die Reaktivität von Einzelstrang- und Doppelstrang-spezifischen Nucleasen in der vorhergesagten Hairpin-Region zwischen Positionen G16 / C26 war gering, so daß hier vermutlich eine kompakte Tertiärstruktur vorliegt.

### Reziproke Bindungsspezifitäten

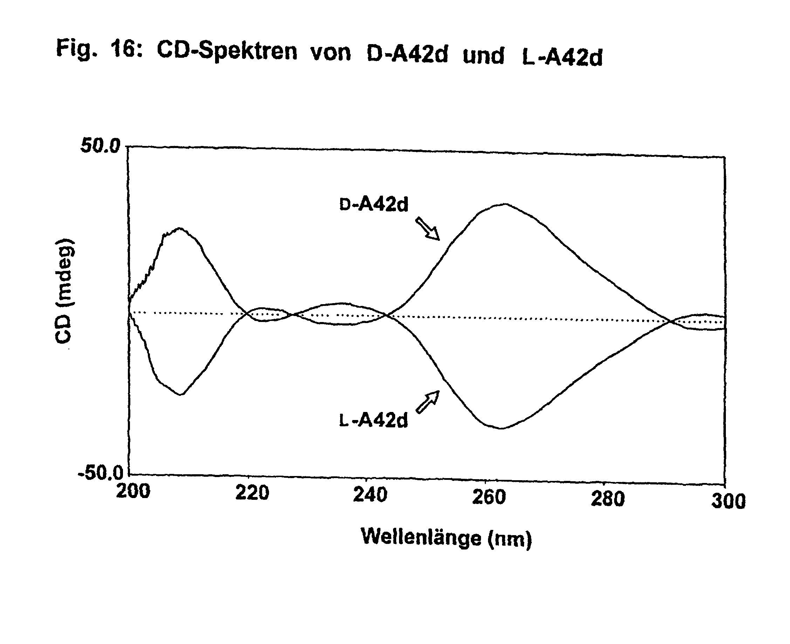

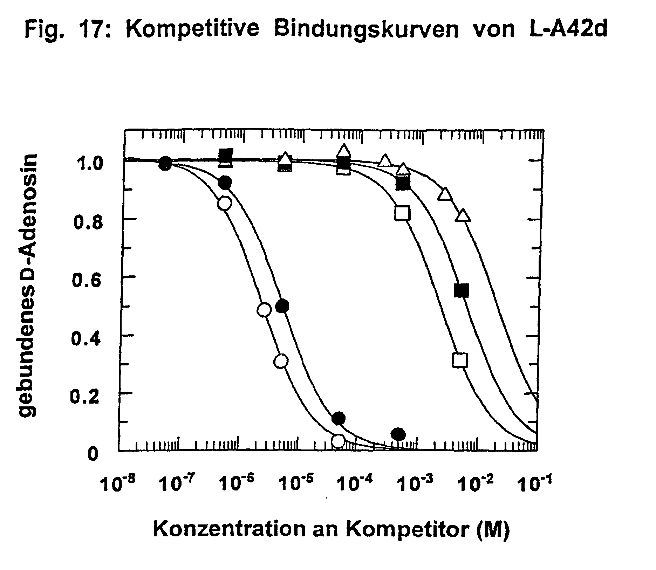

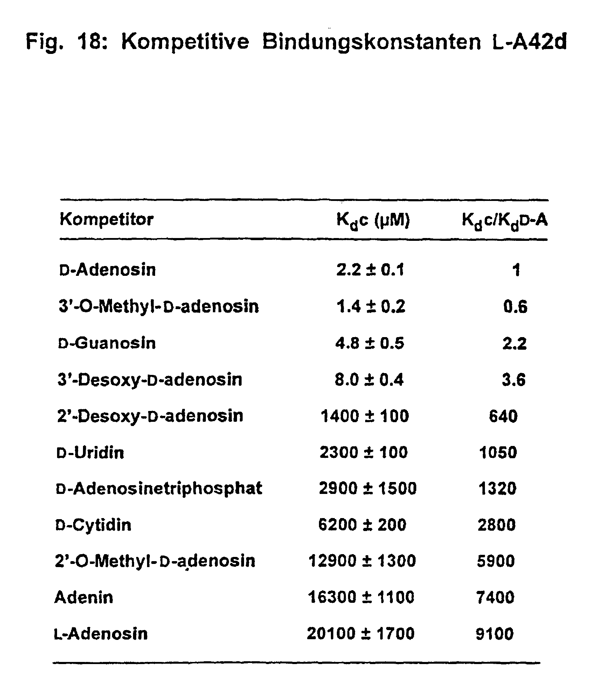

Nach der Identifizierung von D-A42d wurde, wie in Beispiel 1 beschrieben, ein L-Oligoribonucleotid mit identischer Nucleotidsequenz (Fig. 13) durch chemische Festphasensynthese präpariert. Die stereochemische Reinheit des L-Oligoribonucleotids wurde durch Circular-Dichroismus-Spektrometrie evaluiert. CD Spektren wurden von 1 A₂₆₀ Oligonucleotid in 0.1 M NaCl und 10 mM Natriumphosphat, pH 7.0 bei 4°C mit einem JASCO J-600 Spectropolarimeter aufgenommen. Die Spektren von D-A42d und L-A42d zeigen gleichgroße und entgegengesetzte optische Rotationen (Fig. 16). Verglichen mit der Bindung von D-A42d an L-Adenosin, zeigte der spiegelbildliche Ligand L-A42d identische Affinität für D-Adenosin (*K*_{d} von 1.8 ± 0.1 µM) (Fig. 14). Der Nachweis der chiralen Bindungsspezifitäten konnte durch Kompetition bestätigt werden. Die Affinitäten von D-A42d für D-Adenosin and L-A42d für L-Adenosin liegen bei > 20 mM (Fig. 17 und 18). Die homochiralen Wechselwirkungen sind damit mehr als 9 000-fach schwächer als die heterochiralen Affinitäten. Diese reziproken chiralen Eigenschaften der Liganden zeigen, daß für Nucleinsäuren keine Verletzung der Parität vorliegt und daß die Bindungsaktivitäten ausschließlich von der Nucleotidsequenz bestimmt werden.

### Molekulare Komponenten der Wechselwirkung mit L-A42d

Die Wechselwirkung von L-Oligoribonucleotid und D-Adenosin wurde durch Kompetition mit Nucleosidanaloga charakterisiert (Fig. 17 and 18). Die Kompetitionsexperimente wurden, wie oben beschrieben, durch Gleichgewichtsdialyse in Micro-Dialysekammern bestimmt, wobei 3 µM RNA mit 240 nM Adenosin und steigenden Konzentrationen an Kompetitor äquilibriert wurden. Wie oben beschrieben wurde der Prozentanteil gebundenen Adenosins bestimmt und in der Abwesenheit von Kompetitor auf 1 normalisiert. Die Daten wurden nach Lin und Riggs (J. Mol. Biol. 72 (1972), 671-690) einer Standard-Kompetitionsgleichung mit 1:1 Stöchiometrie von Kompetitor zu RNA angepaßt, wobei eine Dissoziationskonstante von 1.8 µM für die L-A42d-Bindung an D-Adenosin verwendet wurde. Die N-glykosidische Bindung des Nucleosids hat bei der Wechselwirkung eine wichtige Funktion, da D-Guanosin einen *K*_{d}c von 4.8 µM aufweist, während die Kompetitor-Dissoziationskonstanten von D-Uridin and D-Cytidin im millimolaren Bereich liegen. In der Abwesenheit der 2'-Hydroxylgruppe erhöht sich der *K*_{d}c auf 1.4 mM für 2'-Desoxy-D-adenosin und auf 12.9 mM für 2'-O-Methyl-D-adenosin, während die Kompetition mit 3'-Deoxy-D-Adenosin und 3'-O-Methyl-D-adenosin zu einem ähnlichen oder besseren *K*_{d}c führt, als für D-Adenosin erhalten wurde. Neben der N-glycosidischen Bindung scheint daher auch die 2'-Hydroxylgruppe fur die molekulare Wechselwirkung wichtig zu sein.

### Serumstabilität der Adenosin-spezifischen Liganden

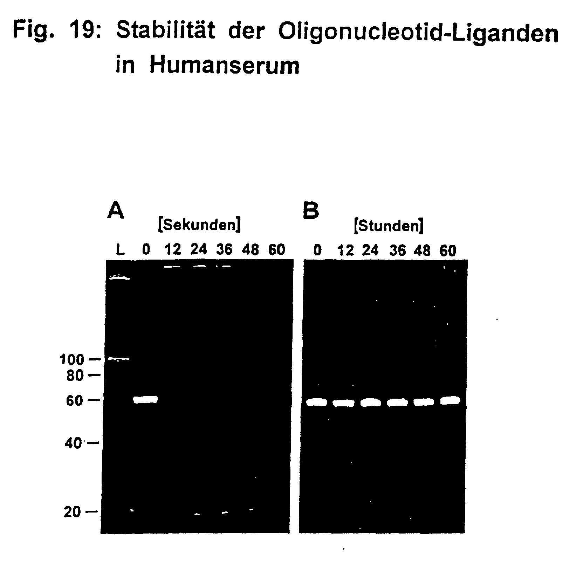

Die D- und L-Oligoribonucleotide wurden bei einer Konzentration von 10 µM in 90 % Humanserum (Sigma, H-1388), das mit 10 mM Natriumphosphat, pH 7.0 gepuffert wurde, inkubiert. Um konstante Volumen- und pH-Bedingungen bei Langzeitversuchen zu erhalten, wurden die Experimente bei Langzeitversuchen in einem Inkubator bei 37° C, 94.5 % Feuchtigkeit und 5 % CO₂ durchgeführt. Entnommene Aliquote wurden mit gleichen Volumina einer Stoplösung (8 M Harnstoff, 50 mM EDTA und 2% SDS) versetzt und in flüssigem Stickstoff eingefroren. Die Proben (55 pmol RNA) wurden auf einem denaturierenden 12% Polyacrylamidgel getrennt. Die Gele wurden mit Ethidiumbromid-Lösung (1 µg/ml) gefärbt und die Banden bei 254 nm sichbar gemacht. Digitalisierte Gelaufnahmen von einem Video-Densitometer wurden mit einem Computerprogramm ausgewertet. Während das D-Oligoribonucleotid D-A42d innerhalb weniger Sekunden in Humanserum abgebaut wird, läßt sich für das L-Oligoribonucleotid L-A42d unter identischen Bedingungen auch nach 60 Stunden kein Abbau nachweisen (Fig. 19).

### Beispiel 3

### Verfahren zur Identifikation von L-Arginin-spezifischen L-Oligoribonucleotiden

### Kopplung von D-Arginin

Das Affinitätsmaterial wurde durch Kopplung von D-Arginin an Epoxy-aktivierte Agarose präpariert. Hierfür wurden 5 g Epoxy-aktivierte Agarose 6B (Pharmacia) mit Wasser und Kopplungspuffer (0.1 M Natriumcarbonat-Puffer, pH 9.5) gewaschen. Die Agarose wurde in 10 mM D-Arginin (Sigma) in Kopplungspuffer and Spurenmengen von L-[4,5-³H]Arginin resuspendiert. Nach Inkubation für 24 Stunden bei Raumtemperatur unter leichtem Schütteln wurde das Material mit Kopplungspuffer und Wasser gewaschen. Die Agarose wurde dann in 1 M Ethanolamin, pH 10.0, für 4 Stunden bei 32°C inkubiert, um verbliebene aktive Gruppen zu blockieren. Die Kapazität des Gels wurde durch Szintillationszählung bestimmt. Mit Ausnahme des Kopplungsschritts wurde das Vorsäulenmaterial identisch präpariert.

### Identifizierung eines D-Arginin-Bindungsmotivs

Eine kombinatorische D-RNA Bibliothek mit 10¹⁴ Zufallssequenzen wurde durch *in vitro*-Transkription mit T7-RNA-Polymerase aus einem Gemisch von DNA Molekülen gewonnen, die eine 50-Nucleotide lange Region mit Zufallssequenzen enthielten. Begrenzt wurde die Region durch 20 Nucleotide lange DNA-Bereiche mit vorgegebener Sequenz (vgl. Beispiel 2). Elf Selektionszyklen wurden, wie in Beispiel 2 beschrieben durchgeführt, wobei das Affinitätsmaterial mit 1 mM D-Arginin beladen war und für die Chromatographie eine Lösung von 320 mM NaCl, 50 mM Tris-HCl, pH 7.5. und 5 mM MgCl₂ als Selektionspuffer eingesetzt wurde. RNA-Varianten mit Affinität zum Säulenmaterial wurden in allen Zyklen durch Bindung an das Agarose-Vorsäulematerial entfernt. D-Argininspezifische RNA-Moleküle wurden mit 4 Säulenvolumina einer 15 mM D-Arginin-Lösung in Selektionspuffer eluiert. Um die Stereoselektivität der Liganden zu erhöhen, wurde die Affinitätssäule nach dem fünften Zyklus zunächst mit L-Arginin gewaschen und dann erst die noch gebundenen RNA-Moleküle mit D-Arginin eluiert. Nach elf Selektionszyklen wurden die RNA-Moleküle revers transkribiert, kloniert und sequenziert.

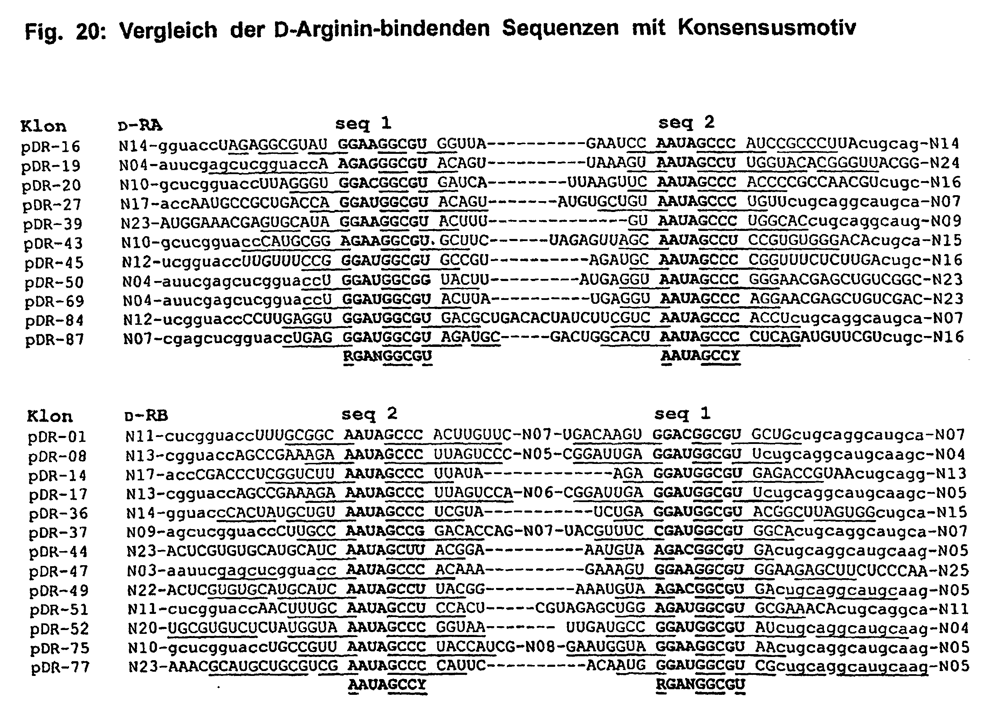

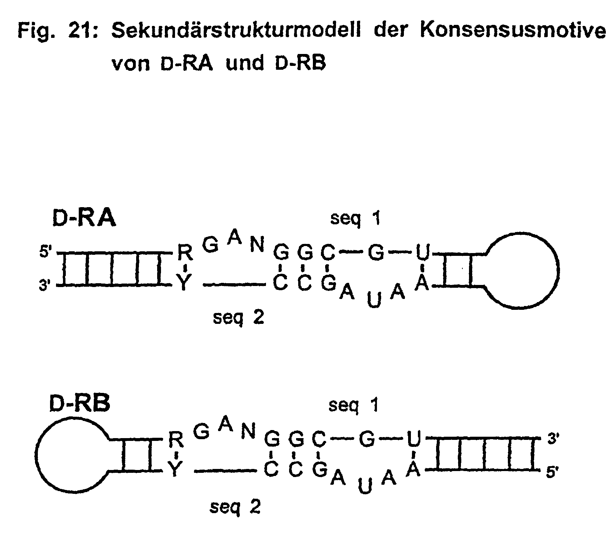

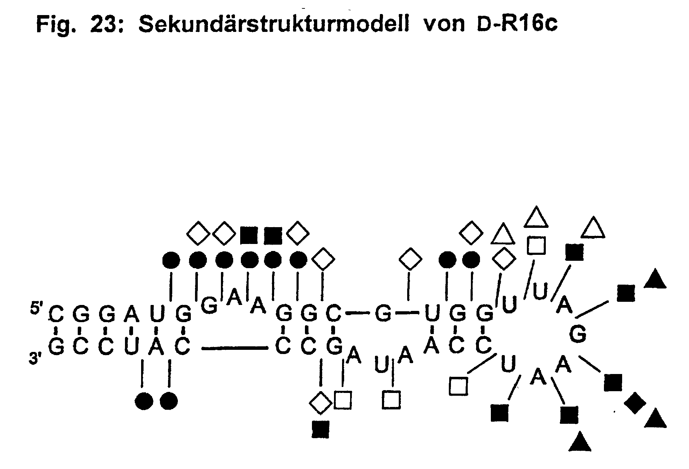

Bei der Sequenzierung von 55 Klonen konnten 41 verschiedene Sequenzen gefunden werden. Der Sequenzvergleich (Fig. 20) ergab zwei hochkonservierte Sequenzelemente, die in fast 60% der Sequenzen vorhanden war: Ein 9 Nucleotide langes Motiv (seq 1) und ein 8 Nucleotide langes Motiv (seq 2). In 11 Sequenzen lag seq 1 näher am 5'-Terminus als seq2, während in 13 Sequenzen die Konsensusmotive in umgekehrter Reihenfolge vorlagen. Die Sequenzen mit den Konsensusmotiven konnten mit dem Algorithmus von Zuker (a.a.O) in ein gemeinsames Sekundärstrukturmodell gefaltet werden (Fig. 21). Ausgehend von diesem Modell wurden von D-R16 verkürzte Varianten durch chemische RNA-Synthese präpariert. Die Variante D-R16c, ein 38 Nucleotide langes RNA-Molekül (Fig. 23), enthält die wichtigen Strukturelemente für die spezifische Bindung von D-Arginin (siehe unten).

### Charakterisierung der L-Arginin-spezifischen RNA-Moleküle

Die Strukturanalyse von D-R16c durch enzymatischen Verdau mit den Nucleasen T1, T2, S1, and V1 (Fig. 22) steht in Übereinstimmung mit dem Sekundärstrukturmodell (Fig. 23). Die Phosphodiesterbindungen im Hairpin-Loop und den internen Loops konnten durch Einzelstrang-spezifische Nukleasen gespalten werden. Während die Nucleotide im internen AUA-Loop und im GAN-Bulge hochgradig konserviert sind, konnten für die Nucleotide in der Hairpin-Struktur, die die Sequenzelemente seq1 und seq2 miteinander verbindet, keine konservierten Positionen gefunden werden. Die Hairpin-Region scheint daher nicht direkt an der Bindung von Arginin beteiligt zu sein.

### Reziproke Bindungsspezifitäten

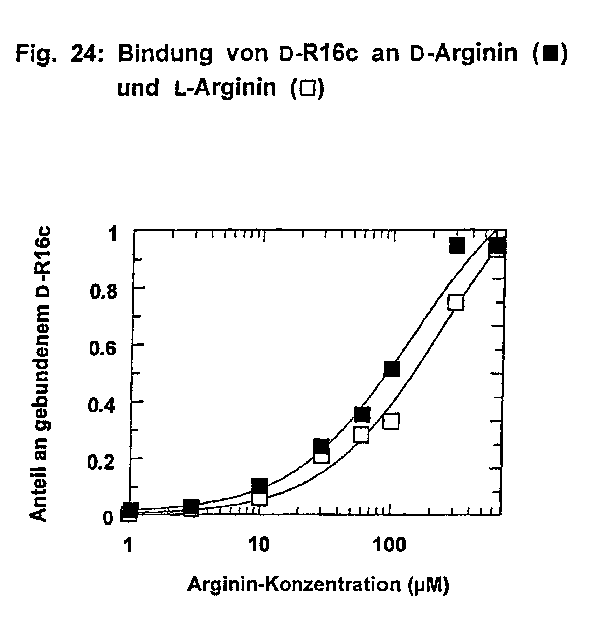

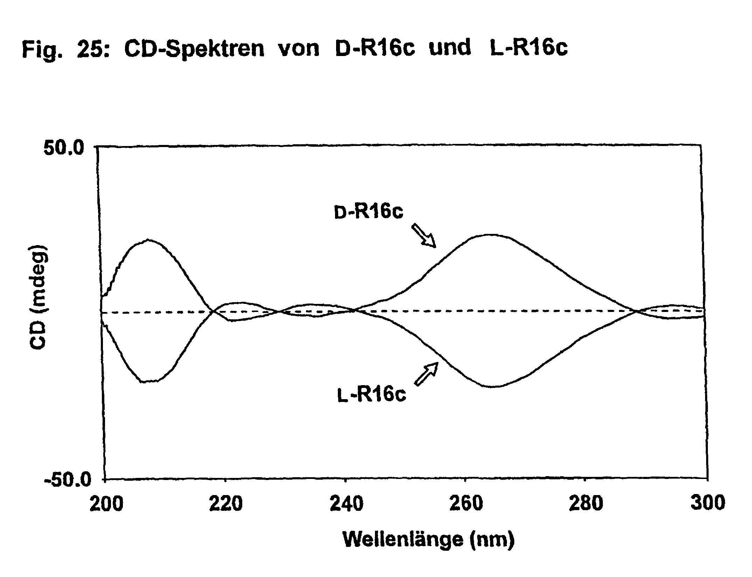

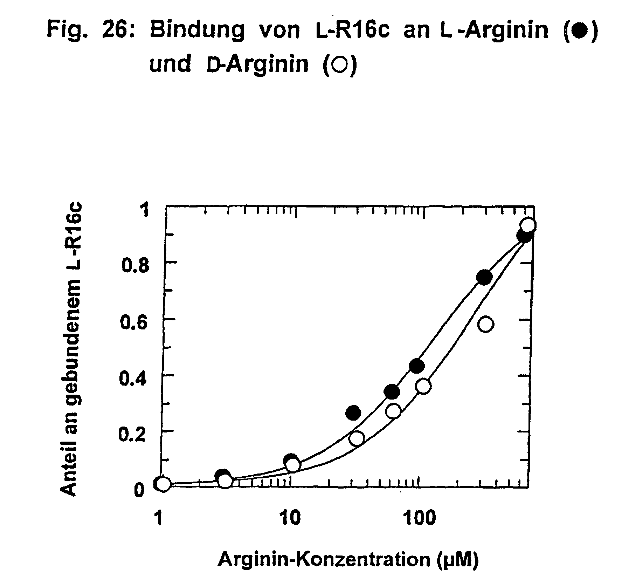

Das L-Enantiomere von D-R16c (als L-R16c bezeichnet) wurde durch chemische Festphasensynthese erhalten (vgl. Beispiel 1). Bei der Charakterisierung von D-R16c und L-R16c durch Circular-Dichroismus-Spektrometrie wurden erwartungsgemäß spiegelbildliche Spektren erhalten (Fig. 25, vgl. Beispiel 2). Die Affinität der Oligonucleotid-Liganden für D- bzw. L-Arginin wurde, wie in Beispiel 2 beschrieben, durch Gleichgewichtsdialyse in Micro-Dialysekammem ermittelt. Steigende Mengen an L-[4,5-³H]Arginin (ICN) oder D-[2,3-³H]Arginin (DuPont, NEN, Kundensynthese) wurden in Bindungspuffer (50 mM NaCl, 50 mM Tris-HCI, pH 7.5, 5 mM MgCl₂) mit 10 µM der D- oder L-RNA äquilibiert. D-R16c zeigte eine Dissoziationskonstante (K_{d}) von 135 ± 25 µM für die Wechselwirkung mit D-Arginin (Fig. 24). L-R16c konnte L-Arginin mit einem nicht unterscheidbaren K_{d} von 129 ± 18 µM binden (Fig. 26). Die Bindung von D-R16c an D-Arginin war stereoselectiv mit einer 1.7-fachen Präferenz von D-Arginin über L-Arginin (Fig. 24). L-R16c zeigte eine 1.8-fache Stereoseleclivität für L-Arginin (Fig. 26). Diese Ergebnisse verdeutlichen die reziprok-identischen Bindungseigenschaften der Oligonucleotid-Enantiomere.

### Molekulare Komponenten der Wechselwirkung mit L-R16c

Um die spezifischen Komponenten zu charakterisieren, die an der Bindung beteiligt sind, wurden kompetitive Bindungsexperimente durchgeführt (vgl. Beispiel 2). Steigende Mengen an Kompetitor wurden zu 10 µM L-[4,5-³H]Arginin gegeben und gegen 10 µM L-R16c äquilibriert. Der Anteil an gebundenem L-Arginin wurde aus dem Verhältnis von gebundenem Arginin mit Kompetitor zu gebundenem Arginin ohne Kompetitor bestimmt. Die Daten wurden nach Lin und Riggs (a.a.O.) an eine Standardbindungsgleichung angepaßt, wobei ein K_{d} von 130 µM für die Binding von L-R16c an L-Arginin verwendet wurde. Mit L-Arginin als Kompetitor wurde für L-R16c eine kompetitive Dissoziationskonstante (K_{d}c) von 60 ± 10 µM ermittelt. Die Bindungsaffinität von L-R16c für L-Lysin (K_{d}c = 270 ± 50 µM) war etwa 40-fach größer als für D-Lysin (K_{d}c > 10 mM). Das Ergebnis deutet darauf hin, daß die α-Carboxyl-Gruppe an der Bindung beteiligt ist. Agmatin, ein Arginin-Analogon, dem die α-Carboxyl-Gruppe fehlt, zeigte eine kompetitive Dissoziationskonstante von 270 ± 70 µM. Dieses Resultat legt nahe, daß die L-RNA auch mit der Guanidinium-Seitenkette in Wechselwirkung tritt. Die simultane Bindung der Seitenkette und der α-Carboxylgruppe führt zur L-Präferenz der Bindungsregion. Die relativ starke Bindung an die Seitenkette erklärt die niedrige Stereoselektivität von L-R16c für L-Arginin.

### Bindung von L-R16c an ein HIV-Tat-Peptid

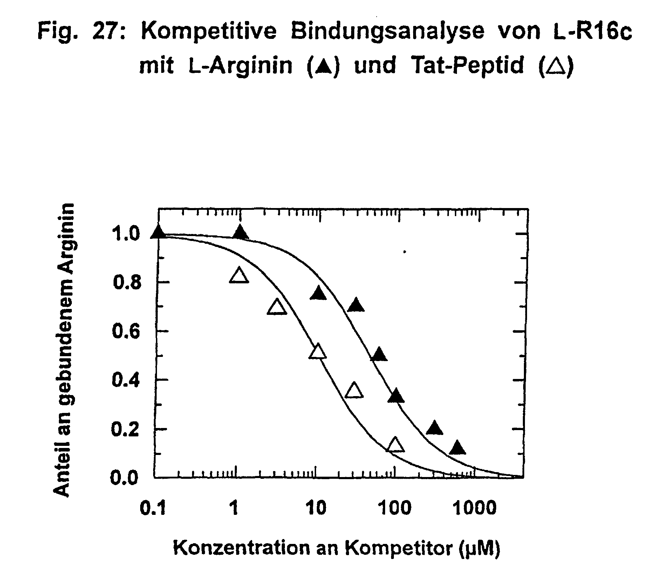

Die Arginin-spezifischen Bindungseigenschaften von L-R16c wurden genutzt, um die Wechselwirkung mit Peptiden zu testen. Kompetitive Bindungsexperimente wurden mit einem Peptid durchgeführt, das die Sequenz YGRKKRRQRRRP aus dem HIV-Tat-Protein trägt. In Abweichung zu den oben beschriebenen Kompetitionsanalysen wurde eine Dialysemembran mit einer Molekulargewichts-Ausschlußgrenze von 8000 eingesetzt. Die Affinität von L-R16c für das Tat-Peptid (K_{d}c = 26 ± 5 µM) war um den Faktor zwei höher als die Affinität für L-Arginin (Fig. 27).

### Serumstabilität der Arginin-spezifischen Liganden

Die Stabilität von D-R16c und L-R16c wurde, wie in Beispiel 2 aufgerührt, in Humanserum untersucht. Während D-R16c in weniger als einer Minute nicht mehr nachweisbar war, konnte für L-R16c auch nach Inkubation für 60 Stunden bei 37°C kein Abbau festgestellt werden.

### Beispiel 4

### Verfahren zur iterativen in vitro-Vereinzelung und Sequenzierung

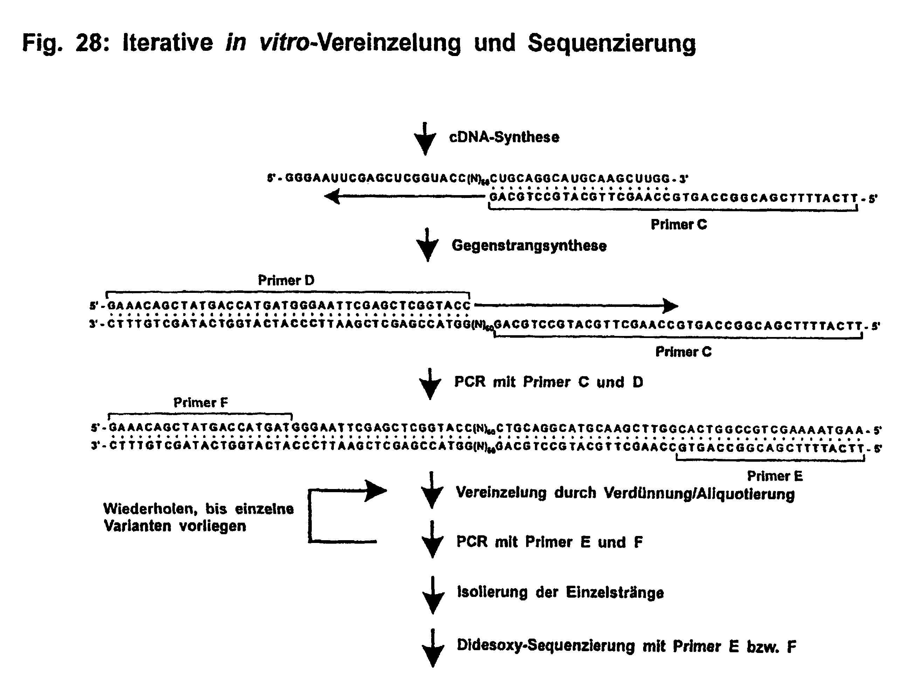

Die in Beispiel 2 beschriebenen RNA-Moleküle aus der zehnten Selektionsrunde wurden erneut in die komplementäre cDNA durch Reverse Transkriptase übersetzt, dann die Gegenstrangsynthese und schließlich die PCR-Amplifikation durchgefürt (Fig. 28). In Abweichung zu Beispiel 2 wurden als Primer Oligonukleotide verwendet, die mit jeweils 20 Nukleotiden über die RNA- bzw. DNA-Matrize hinausragen (Primer C und D, Fig. 28). Diese Verlängerung der PCR-Produkte wurde durchgeführt, um durch Didesoxy-Sequenzierung die randomisierte Region vollständig ermitteln zu können.

Die PCR-Produkte wurden über ein denaturierendes Polyacrylamidgel (7 M Harnstoff) gereinigt. Die DNA-Moleküle wurden aus dem Gel mit Wasser eluiert und durch Gelfiltration entsalzt. Die isolierte Menge an DNA-Produkt wurde durch Absorption bei 260 nm bestimmt. Zur iterativen Vereinzelung wurden die DNA-Moleküle in mehreren Schritten derart verdünnt, daß theoretisch eine Zahl von etwa 100 Molekülen erreicht wurde. Aus dieser Verdünnungstufe konnte durch Aliquotierung eine weitere Verringerung der Anzahl an DNA-Molekülen erreicht werden.

Die pro Aliquot nur noch wenigen DNA-Moleküle wurden durch PCR vermehrt (Primer E und F, Fig. 28). Es wurden mehrere PCR-Reaktionen à 30 Zyklen benötigt, um die DNA-Moleküle so zu amplifizieren, daß sie auf einem Polyacrylamidgel nachweisbar waren (5 pmol). Nach jeder PCR-Reaktion wurde das erhaltene Material durch Ethaholfällung in Gegenwart von Glykogen gereinigt.

Zur Isolierung von Einzelsträngen wurden in erneuten PCR-Reaktion Primer verwendet, die am 5'-Ende phosphoryliert waren (Primer E oder F, Fig. 28). Die 5'-Phosphatgruppe wurde während der Festphasensynthese der Primer eingerührt. Vor den eigentlichen Sequenzierungsreaktionen wurde aus den doppelsträngigen PCR-Produkten Einzelstrang DNA hergestellt, indem der phosphorylierte Strang durch lambda Exonuklease selektiv abgebaut wurde (Higuchi und Ochman, Nucleic Acids Res. 17 (1989), 5865). Die Sequenzierung wurde dann nach dem konventionellen Didesoxynukleotid-Verfahren durchgerührt (Sanger et al., Proc. Natl. Acad. Sci. USA 74 (1977), 5463-5467).

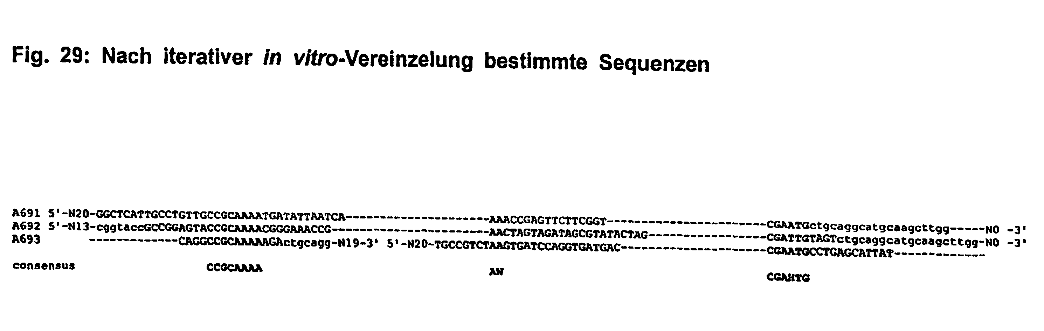

Wenn bei Auswertung der Sequenzgele ersichtlich wurde, daß noch mehrere Varianten vorlagen, wurde das Verdünnungsverfahren solange wiederholt bis die individuelle Sequenz ermittelt werden konnte. Mit diesem Verfahren konnten neben schon bekannten Varianten (Abb. 12) drei neue Varianten identifiziert werden (Abb. 29).

## Patentansprüche

1. Verfahren zur Herstellung von L-Nucleinsäuren mit einer Mindestlänge von 15 Nucleotiden enthaltend L-Adenosin, L-Cytidin, L-Guanosin und L-Uridin, welche an ein in der natürlichen Konfiguration auftretendes Zielmolekül binden, das die folgenden Schritte umfaßt:
(a) Erzeugung einer heterogenen Population von D-Nucleinsäuren;
(b) Inkontaktbringen der Population von Schritt (a) mit der optischen Antipode des Zielmoleküls;
(c) Abtrennen der D-Nucleinsäuren, die nicht mit der optischen Antipode des Zielmoleküls in Wechselwirkung getreten sind;
(d) Sequenzierung der D-Nucleinsäuren, die mit der optischen Antipode des Zielmoleküls in Wechselwirkung getreten sind; und
(e) Synthese von L-Nucleinsäuren, die in ihrer Sequenz mit den in Schritt (d) für die D-Nucleinsäuren ermittelten Sequenzen identisch sind.

2. Verfahren nach Anspruch 1, wobei die optische Antipode des Zielmoleküls als racemisches Gemisch des Zielmoleküls vorliegt.

3. Verfahren nach Anspruch 1 oder 2, wobei im Anschluß an Schritt (c) folgender Schritt eingefügt wird:
(ca) Amplifikatlon der D-Nucleinsäuren, die mit der optischen Antipode des Zielmoleküls in Wechselwirkung getreten sind.

4. Verfahren nach Anspruch 3, wobei die D-Nucleinsäuren der Population von Schritt (a) an ihren 5'- und 3'-Enden Primerbindungsstellen bzw. Komplementärsequenzen zu Primerbindungsstellen aufweisen, die eine Amplifikation der in Schritt (ca) erhaltenen D-Nucleinsäuren durch PCR ermöglichen.

5. Verfahren nach Anspruch 3 oder 4, wobei die D-Nucleinsäure der Population von Schritt (a) an ihren 5'- oder 3'-Enden Bindungsstellen bzw. Komplementärsequenzen zur Bindungsstelle für eine DNA-abhängige RNA-Polymerase aufweisen, die eine in vitro Transkription der in Schritt (ca) erhaltenen cDNAs ermöglichen.

6. Verfahren nach einem der Ansprüche 3 bis 5, wobei während der Amplifikation Nucleotide in die neu zu synthetisierenden Nucleotidstränge eingebaut werden, die an dieser Nucleinsäureposition in den in Schritt (a) vorhandenen, mit dem Zielmolekül wechselwirkenden D-Nucleinsäuren nicht vorkommen.

7. Verfahren nach einem der Ansprüche 3 bis 6, wobei im Anschluß an Schritt (ca) folgender Schritt eingefügt wird:
(cb) Inkontaktbringen der amplifizierten D-Nucleinsäuren mit der optischen Antipode des Zielmoleküls,
an die sich die Schritte (b) und gegebenenfalls (ca) vor der Durchführung des Schrittes (d) anschließen, wobei die Schritte (cb), (b), und gegebenenfalls (ca) in dieser Reihenfolge ein oder mehrere Male wiederholt werden können.

8. Verfahren zur Herstellung von L-Nucleinsäuren mit einer Mindestlänge von 15 Nucleotiden enthaltend L-Adenosin, L-Cytidin, L-Guanosin und L-Uridin, das die folgenden Schritte umfaßt:
(a) Erzeugung einer heterogenen Population von L-Nucleinsäuren;
(b) Inkontaktbringen der Population von Schritt (a) mit dem Zielmolekül;
(c) Abtrennen der L-Nucleinsäuren, die nicht mit dem Zielmolekül in Wechselwirkung getreten sind;
(d) Sequenzierung der L-Nucleinsäuren, die mit dem Zielmolekül in Wechselwirkung getreten sind;
(e) Synthese von L-Nucleinsäuren, die in ihrer Sequenz mit den in Schritt (d) ermittelten Sequenzen identisch sind.

9. Verfahren nach Anspruch 8, wobei zusätzlich im Anschluß an Schritt (c) folgender Schritt eingefügt wird:
(ca) Amplifikation der L-Nucleinsäuren, die mit dem Zielmolekül in Wechselwirkung getreten sind.

10. Verfahren nach Anspruch 9, wobei die L-Nucleinsäuren der Population von Schritt (a) an ihren 5'- und 3'-Enden Primerbindungsstellen bzw. Komplementärsequenzen zu Primerbindungsstellen aufweisen, die eine Amplifikation der in Schritt (ca) erhaltenen L-Nucleinsäuren durch spiegelbildliche PCR ermöglichen.

11. Verfahren nach Anspruch 9 oder 10, wobei bei der Amplifikation Nucleotide in die neu zu synthetisierenden Nucleotidstränge eingebaut werden, die an dieser Nucleotidsäureposition in den in Schritt (a) vorkommenden, mit dem Zielmolekül wechselwirkenden L-Nucleinsäuren nicht vorkommen.

12. Verfahren nach einem der Ansprüche 9 bis 11, wobei im Anschluß an Schritt (ca) folgender Schritt eingefügt wird:
(cb) Inkontaktbringen der amplifizierten L-Nucleinsäuren mit dem Zielmolekül, woran sich die Schritte (b) und gegebenenfalls (ca) vor der Durchführung des Schrittes (d) anschließen, wobei die Schritte (cb), (b) und gegebenenfalls (ca) in dieser Reihenfolge ein oder mehrere Male wiederholt werden können.

13. Verfahren nach einem der Ansprüche 1 bis 12, wobei die Wechselwirkung in einer Bindung besteht.

14. Verfahren nach einem der Ansprüche 1 bis 13, wobei die Wechselwirkung in einer katalytischen Reaktion besteht.

15. Verfahren nach einem der Ansprüche 1 bis 14, wobei die Nucleinsäuren Ribonucleinsäuren sind.

16. Verfahren nach Anspruch 15, wobei die Ribonucleinsäuren Ribozyme sind.

17. Verfahren nach einem der Ansprüche 1 bis 16, wobei das Zielmolekül eine Aminosäure, ein Peptid, ein Polypeptid oder ein aus mehreren Polypeptiden zusammengesetztes Protein ist.

18. Verfahren nach einem der Ansprüche 1 bis 17, wobei das Zielmolekül eine einzelsträngige RNA, eine doppelsträngige RNA, eine einzelsträngige DNA oder eine doppelsträngige DNA oder eine Kombination daraus ist.

19. Verfahren nach einem der Ansprüche 1 bis 17, wobei das Zielmolekül ein Antibiotikum oder ein anderes, pharmazeutisch wirksames Substrat ist oder dessen Vorstufe ist.

20. Verfahren nach einem der Ansprüche 1 bis 17, wobei das Zielmolekül ein Zuckermolekül, beispielsweise ein unverzweigter oder verzweigter Polyzucker ist.

21. Verfahren nach einem der Ansprüche 1 bis 20, wobei die Synthese der L-Nucleinsäuren in Schritt (e) auf chemischem oder enzymatischem Wege erfoigt.

22. Verfahren nach Anspruch 21, wobei die chemische Synthese folgende Schritte umfaßt
(ea) Synthese von L-Nucleosiden;
(eb) Synthese von geschützten L-Nucleosidphosphoramiditen; und
(ec) Festphasensynthese der L-Nucleinsäuren am Automaten.

23. L-Nucleinsäure mit einer Mindestlänge von 15 Nucleotiden enthaltend L-Adenosin, L-Cytidin, L-Guanosin und L-Uridin, die spezifisch mit einem in den Ansprüchen 1 bis 19 definierten Zielmolekül in Wechselwirkung tritt.

24. L-Nucleinsäure nach Anspruch 23, die eine L-Ribonucleinsäure ist.

25. L-Nucleinsäure nach Anspruch 24, die ein Ribozym ist.

26. Verwendung der nach dem Verfahren von einem der Ansprüche 1 bis 22 in Schritt (c) und/oder Schritt (ca) erhältlichen D-Nucleinsäuren mit einer Mindestlänge von 15 Nucleotiden als Matrize zur Herstellung einer L-Nucleinsäure mit identischer Nucleinsäuresequenz.

27. Arzneimittel, enthaltend eine nach dem Verfahren von einem der Ansprüche 1 bis 22 erhältliche L-Nucleinsäure oder eine L-Nucleinsäure nach einem der Ansprüche 23 bis 25, gegebenenfalls in Kombination mit einem pharmazeutisch verträglichen Träger.

28. Kit oder diagnostisches Mittel, enthaltend eine nach dem Verfahren von einem der Ansprüche 1 bis 22 erhältliche L-Nucteinsäure oder eine L-Nucleinsäure nach einem der Ansprüche 23 bis 25.

29. Verwendung der nach dem Verfahren von einem der Ansprüche 1 bis 22 erhältlichen L-Nucleinsäuren oder eine L-Nucleinsäure nach einem der Ansprüche 23 bis 25 als Biosensoren, Herbizide, Zusatzstoffe für Nahrungsmittel, für analytische Verfahren oder für kosmetische Anwendungen.

## Claims

1. A process for producing L-nucleic acids having a minimal length of 15 nucleotides containing L-adenosine, L-cytidine, L-guanosine and L-uridine which bind to a target molecule occuring in the natural configuration, comprising the following steps:
(a) producing a heterogenic population of D-nucleic acids;
(b) contacting the population of step (a) with the optical antipode of the target molecule;
(c) separating the D-nucleic acids which have not interacted with the optical antipode of the target molecule;
(d) sequencing the D-nucleic acids which have interacted with the optical antipode of the target molecule; and
(e) synthesis of L-nucleic acids which, with respect to their sequence, are identical to the D-nucleic acids found in step (d).

2. The process according to claim 1, wherein the optical antipode of the target molecule is present in the form of a racemic mixture of the target molecule.

3. The process according to claim 1 or 2, wherein subsequent to step (c) the following step is introduced:
(ca) amplification of the D-nucleic acids which have interacted with the optical antipode of the target molecule.

4. The process according to claim 3, wherein the D-nucleic acids of the population of step (a) have, at their 5'- and 3'-ends, primer binding sites or complementary sequences to primer binding sites which render an amplification of the D-nucleic acids of step (ca) by PCR possible.

5. The process according to claim 3 or 4, wherein the D-nucleic acid of the population of step (a), at their 5'- or 3'-ends, has binding sites or complementary sequences to the binding site for a DNA-depending RNA polymerase which render an in vitro transcription of the cDNAs obtained in step (ca) possible.

6. The process according to any one of claims 3 to 5, wherein during the amplification, nucleotides are integrated into the nucleotide strains which are to be newly synthesised, the nucleotides not being present at this position of the nucleic acid in the D-nucleic acids which are present in step (a) and which interact with the target molecule.

7. The process according to any one of claims 3 to 6, wherein subsequent to step (ca) the following step is carried out:
(cb) contacting the amplified D-nucleic acids with the optical antipode of the target molecule,
the steps (b) and optionally (ca) following before the carrying out step (d), wherein the steps (cb), (b) and optionally (ca) can be repeated in this order once or several times.

8. A process for the production of L-nucleic acids having a minimal length of 15 nucleotides containing L-adenosine, L-cytidine, L-guanosine and L-uridine, comprising the following steps:
(a) producing a heterogenic population of L-nucleic acids;
(b) contacting the population of step (a) with the target molecule;
(c) separating the L-nucleic acids which have not interacted with the target molecule;
(d) sequencing the L-nucleic acids which have interacted with the target molecule;
(e) synthesis of L-nucleic acids which, with respect to their sequence, are identical with the sequences found in step (d).

9. The process according to claim 8, wherein subsequent to step (c) the following step is added:
(ca) amplification of the L-nucleic acids which have interacted with the target molecule.

10. The process according to claim 9, wherein the L-nucleic acids of the population of step (a) have, at their 5'- and 3' ends, primer binding sites or complementary sequences to primer binding sites which render an amplification of the L-nucleic acids obtained in step (ca) by mirror-image PCR possible.

11. The process according to claim 9 or 10, wherein, during the amplification, nucleotides are integrated into the nucleotide strains which are to be newly synthesised, the nucleotides not being present at this position of the nucleotide acid in the L-nucleic acids which are present in step (a) and which interact with the target molecule.

12. The process according to any one of claims 9 to 11, wherein subsequent to step (ca) the following step is carried out:
(cb) contacting the amplified L-nucleic acids with the target molecule, followed by steps (b) and optionally (ca) prior to the carrying out step (d), wherein the steps (cb), (b) and optionally (ca) can be repeated in this order once or several times.

13. The process according to any one of claims 1 to 12, wherein the interaction is a binding.

14. The process according to any one of claims 1 to 13, wherein the interaction is a catalytic reaction.

15. The process according to any one of claims 1 to 14, wherein the nucleic acids are ribonucleic acids.

16. The process according to claim 15, wherein the ribonucleic acids are ribozymes.

17. The process according to any one of claims 1 to 16, wherein the target molecule is an amino acid, a peptide, a polypeptide or a protein composed of one or more polypeptides.

18. The process according to any one of claims 1 to 17, wherein the target molecule is a single-stranded RNA, a double-stranded RNA, a single-stranded DNA or a double-stranded DNA or a combination thereof.

19. The process according to any one of claims 1 to 17, wherein the target molecule is an antibiotic or another, pharmaceutically active substrate or a precursor thereof.

20. The process according to any one of claims 1 to 17, wherein the target molecule is a sugar molecule, for example an unbranched or a branched polysaccharide.

21. The process according to any one of claims 1 to 20, wherein the synthesis of L-nucleic acids in step (e) is carried out in a chemical or enzymatic manner.

22. The process according to claim 21, wherein the chemical synthesis comprises the following steps
(ea) synthesis of L-nucleosides;
(eb) synthesis of protected L-nucleoside phosphoramidites; and
(ec) solid phase synthesis of the L-nucleic acids in the automat.

23. An L-nucleic acid having a minimal length of 15 nucleotides containing L-adenosine, L-cytidine, L-guanosine and L-uridine, which interacts specifically with one of the target molecules defined in claims 1 to 19.

24. The L-nucleic acid according to claim 23 which is an L-ribonucleic acid.

25. The L-nucleic acid according to claim 24, which is a ribozyme.

26. Use of the D-nucleic acids obtainable according to the process of any one of claims 1 to 22 in step (c) and/or step (ca), having a minimal length of 15 nucleotides as a template for the production of an L-nucleic acid with an identical nucleic acid sequence.

27. A pharmaceutical composition containing an L-nucleic acid obtainable according to the process of any one of claims 1 to 22 or an L-nucleic acid according to any one of claims 23 to 25, optionally in combination with a pharmaceutically acceptable carrier.

28. A kit or diagnostic composition containing an L-nucleic acid obtainable according to the process of any one of claims 1 to 22 or an L-nucleic acid according to any one of claims 23 to 25.

29. Use of the L-nucleic acids obtainable according to the process of any one of claims 1 to 22 or of an L-nucleic acid according to any one of claims 23 to 25 as biosensors, herbicides, additives for foodstuffs, for analytic processes or for cosmetic applications.

## Revendications

1. Procédé de préparation d'acides L-nucléiques ayant une longueur d'au moins 15 nucléotides contenant de la L-adénosine, de la L-cytidine, de la L-guanosine et de la L-uridine, qui se lient à une molécule cible se présentant dans la configuration naturelle, comprenant les étapes suivantes:
(a) production d'une population hétérogène d'acides D-nucléiques;
(b) mise en contact de la population de l'étape (a) avec l'antipode optique de la molécule cible;
(c) séparation des acides D-nucléiques n'ayant pas subi d'interaction avec l'antipode optique de la molécule cible;
(d) séquençage des acides D-nucléiques ayant subi une interaction avec l'antipode optique de la molécule cible; et
(e) synthèse d'acides L-nucléiques identiques par la séquence aux séquences déterminées dans l'étape (d) pour les acides D-nucléiques.

2. Procédé selon la revendication 1, dans lequel l'antipode optique de la molécule cible se présente sous forme d'un mélange racémique de la molécule cible.

3. Procédé selon la revendication 1 ou 2, dans lequel, après l'étape (c), on introduit l'étape suivante:
(ca) amplification des acides D-nucléiques ayant subi une interaction avec l'antipode optique de la molécule cible.

4. Procédé selon la revendication 3, dans lequel les acides D-nucléiques de la population de l'étape (a) présentent aux extrémités 5' et 3' des sites de liaison d'amorces ou des séquences complémentaires à des sites de liaison d'amorces permettant l'amplification par PCR des acides D-nucléiques obtenus dans l'étape (ca).

5. Procédé selon la revendication 3 ou 4, dans lequel les acides D-nucléiques de la population de l'étape (a) présentent à l'extrémité 5' ou 3' des sites de liaison ou des séquences complémentaires à des sites de liaison pour une ARN-polymérase dépendante de l'ADN, permettant une transcription *in vitro* des ADNc obtenus dans l'étape (ca).

6. Procédé selon l'une des revendications 3 à 5, dans lequel, pendant l'amplification, on incorpore dans les brins de nucléotides qui vont être nouvellement synthétisés des nucléotides qui n'existent pas dans cette position d'acide nucléique dans les acides D-nucléiques présents dans l'étape (a) et réagissant avec la molécule cible.

7. Procédé selon l'une des revendications 3 à 6, dans lequel, après l'étape (ca), on introduit l'étape suivante:
(cb) mise en contact des acides D-nucléiques amplifiés avec l'antipode optique de la molécule cible,
puis on effectue les étapes (b) et éventuellement (ca) avant d'effectuer l'étape (d), les étapes (cb), (b) et éventuellement (ca) pouvant être répétées dans cet ordre une ou plusieurs fois.

8. Procédé de préparation d'acides L-nucléiques ayant une longueur d'au moins 15 nucléotides contenant de la L-adénosine, de la L-cytidine, de la L-guanosine et de la L-uridine, comprenant les étapes suivantes:
(a) production d'une population hétérogène d'acides L-nucléiques;
(b) mise en contact de la population de l'étape (a) avec'la molécule cible;
(c) séparation des acides L-nucléiques n'ayant pas subi d'interaction avec la molécule cible;
(d) séquençage des acides L-nucléiques ayant subi une interaction avec la molécule cible; et
(e) synthèse d'acides L-nucléiques identiques par la séquence aux séquences déterminées dans l'étape (d).

9. Procédé selon la revendication 8, dans lequel, après l'étape (c), on introduit en outre l'étape suivante:
(ca) amplification des acides L-nucléiques ayant subi une interaction avec la molécule cible.

10. Procédé selon la revendication 9, dans lequel les acides L-nucléiques de la population de l'étape (a) présentent aux extrémités 5' et 3' des sites de liaison d'amorces ou des séquences complémentaires à des sites de liaison d'amorces permettant l'amplification par PCR inversée des acides L-nucléiques obtenus dans l'étape (ca).

11. Procédé selon la revendication 9 ou 10, dans lequel, pendant l'amplification, on incorpore dans les brins de nucléotides qui vont être nouvellement synthétisés des nucléotides qui n'existent pas dans cette position d'acide nucléique dans les acides L-nucléiques présents dans l'étape (a) et réagissant avec la molécule cible.

12. Procédé selon l'une des revendications 9 à 11, dans lequel, après l'étape (ca), on introduit l'étape suivante:
(cb) mise en contact des acides L-nucléiques amplifiés avec la molécule cible,
puis on effectue les étapes (b) et éventuellement (ca) avant d'effectuer l'étape (d), les étapes (cb), (b) et éventuellement (ca) pouvant être répétées dans cet ordre une ou plusieurs fois.

13. Procédé selon l'une des revendications 1 à 12, dans lequel l'interaction consiste en une liaison.

14. Procédé selon l'une des revendications 1 à 13, dans lequel l'interaction consiste en une réaction catalytique.

15. Procédé selon l'une des revendications 1 à 14, dans lequel les acides nucléiques sont des acides ribonucléiques.

16. Procédé selon la revendication 15, dans lequel les acides ribonucléiques sont des ribozymes.

17. Procédé selon l'une des revendications 1 à 16, dans lequel la molécule cible est un aminoacide, un peptide, un polypeptide ou une protéine composée de plusieurs polypeptides.

18. Procédé selon l'une des revendications 1 à 17, dans lequel la molécule cible est un ARN simple brin, un ARN double brin, un ADN simple brin ou un ADN double brin ou une de leurs combinaisons.

19. Procédé selon l'une des revendications 1 à 17, dans lequel la molécule cible est un antibiotique ou un autre substrat à activité pharmaceutique ou un de ses précurseurs.

20. Procédé selon l'une des revendications 1 à 17, dans lequel la molécule cible est une molécule de sucre, par exemple un polysaccharide linéaire ou ramifié.

21. Procédé selon l'une des revendications 1, à 20, dans lequel la synthèse des acides L-nucléiques dans l'étape (e) s'effectue par voie chimique ou enzymatique.

22. Procédé selon la revendication 21, dans lequel la synthèse chimique comprend les étapes suivantes:
(ea) synthèse de L-nucléosides;
(eb) synthèse de L-nucléoside-phosphoramidites protégés; et
(ec) synthèse en phase solide des acides L-nucléiques dans des appareils automatiques.

23. Acide L-nucléique ayant une longueur d'au moins 15 nucléotides contenant de la L-adénosine, de la L-cytidine, de la L-guanosine et de la L-uridine, qui entre en interaction de façon spécifique avec une molécule cible définie dans les revendications 1 à 19.

24. Acide L-nucléique selon la revendication 23, qui est un acide L-ribonucléique.

25. Acide L-nucléique selon la revendication 24, qui est un ribozyme.

26. Utilisation des acides D-nucléiques ayant une longueur d'au moins 15 nucléotides pouvant être obtenus par le procédé de l'une des revendications 1 à 22 dans l'étape (c) et/ou l'étape (ca) comme matrices pour la préparation d'un acide L-nucléique ayant la même séquence d'acide nucléique.

27. Médicament contenant un acide L-nucléique pouvant être obtenu par le procédé de l'une des revendications 1 à 22 ou un acide L-nucléique selon l'une des revendications 23 à 25, éventuellement en combinaison avec un support pharmaceutiquement acceptable.

28. Trousse ou agent diagnostic contenant un acide L-nucléique pouvant être obtenu par le procédé de l'une des revendications 1 à 22 ou un acide L-nucléique selon l'une des revendications 23 à 25.

29. Utilisation des acides L-nucléiques pouvant être obtenus par le procédé de l'une des revendications 1 à 22 ou d'un acide L-nucléique selon l'une des revendications 23 à 25 comme biocapteurs, comme herbicides, comme additifs alimentaires, pour des procédés d'analyse ou pour des applications cosmétiques.
